(19) Europäisches Patentamt
European Patent Office
Office européen des brevets

(11) Numéro de publication: **0 365 397 B1**

(12) **FASCICULE DE BREVET EUROPEEN**

(45) Date de publication de fascicule du brevet: **28.09.94**

(51) Int. Cl.[5]: **C07H 15/203**, A61K 31/70, C07H 13/04, C07C 321/26, C07C 333/04

(21) Numéro de dépôt: **89402804.2**

(22) Date de dépôt: **11.10.89**

# (54) Nouveaux beta-D-phényl-thioxylosides, leur procédé de préparation et leur utilisation en thérapeutique.

(30) Priorité: **18.10.88 FR 8813688**
**21.06.89 FR 8908253**

(43) Date de publication de la demande:
**25.04.90 Bulletin 90/17**

(45) Mention de la délivrance du brevet:
**28.09.94 Bulletin 94/39**

(84) Etats contractants désignés:
**AT BE CH DE ES FR GB GR IT LI LU NL SE**

(56) Documents cités:

| | |
|---|---|
| **EP-A- 51 023** | **EP-A- 367 671** |
| **EP-A- 0 008 881** | **EP-A- 0 118 676** |
| **EP-A- 0 133 103** | **EP-A- 0 224 849** |
| **EP-A- 0 290 321** | **US-A- 4 584 012** |

**The Journal of Organic Chemistry vol 31, no. 3, Mars 1966, pages 813-816; B Urbas et al.:"Synthesis of purine and pyrimidine nucleosides of thiopentoses".**

(73) Titulaire: **FOURNIER INDUSTRIE ET SANTE**
**38, avenue Hoche**
**F-75008 Paris (FR)**

(72) Inventeur: **Samreth, Soth**
**2, rue de la deuxième Escadre**
**F-21600 Longvic (FR)**
Inventeur: **Millet, Jean**
**15, rue du Tremblois**
**Corcelles les Citeaux**
**F-21910 Saulon la Rue (FR)**
Inventeur: **Bellamy, François**
**Rue Basse Cédex 11**
**Saulon la Rue**
**F-21910 Saulon la Chapelle (FR)**
Inventeur: **Bajgrowicz, Jerzy**
**2, promenade du Rhône**
**F-21000 Dijon (FR)**
Inventeur: **Barberousse, Véronique**
**15, boulevard Eugène Spuller**
**F-21000 Dijon (FR)**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).



Rank Xerox (UK) Business Services
(3.10/3.09/3.3.3)

**Chemische Berichte, vol. 93, no, 11, 1960, pages 2705-2712; H Zinner et al.:"Alkylthio-alpha-D-ribofuranoside und ihre überfahrung in die acetobrom-D-ribofuranose".**

Inventeur: **Renaut, Patrice**
**3, ruelle de Plombières**
**Hauteville - Lès Dijon**
**F-21121 Fontaine lès Dijon (FR)**

(74) Mandataire: **Clisci, Serge et al**
**S.A. FEDIT-LORIOT**
**CONSEILS EN PROPRIETE INDUSTRIELLE**
**38, avenue Hoche**
**F-75008 Paris (FR)**

## Description

La présente invention concerne, en tant que produits industriels nouveaux, les composés β-D-phénylthioxylosides de formule I ci-dessous. Elle concerne également leur procédé de préparation et leur utilisation en thérapeutique, en tant qu'agents antithrombotiques, notamment antithrombotiques veineux.

On a déjà proposé dans EP-B-OO51023 des dérivés de benzoyl-phényl-osides et d'α-hydroxybenzyl-phénylosides en tant qu'agents anti-ulcéreux, anti-agrégants plaquettaires, antithrombotiques et oxygénateurs cérébraux.

On connait également de EP-A-O1331O3 des benzyl-phénylosides utiles en tant qu'agents hypocholestérolémiants et hypolipidémiants, certains de ces composés, en particulier le produit de l'exemple 1, présentant en outre des effets antithrombotiques.

On connait de EP-A-0 290 321 (date de publication : 09.11.1988) des composés β-D-thioxylosides qui sont utiles en tant qu'agents antithrombotiques et qui répondent à la formule

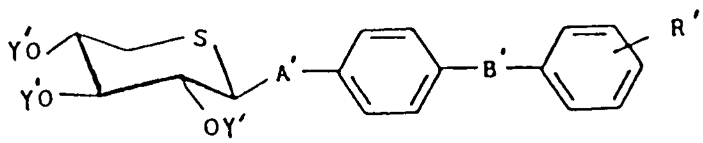

dans laquelle :

- R' représente un atome d'hydrogène, un atome d'halogène, un groupe nitro ou un groupe cyano,
- A' représente l'atome soufre ou l'atome d'oxygène,
- B' représente un groupe $CH_2$, CHOH ou CO, et
- Y' représente l'atome d'hydrogène ou un groupe acyle.

On connaît aussi de EP-A-0 118 676 des composés β-D-xylosides substitués en position 1 du cycle xylopyranosyle par un groupe (4-carboxyphényl)thio ou ZS- où Z est alkyle en $C_4$-$C_{25}$, alcényle en $C_3$-$C_{25}$ ou alcynyle en $C_3$-$C_{25}$. Ces composés sont présentés comme étant utiles en thérapeutique en tant qu'agents anti-ulcer, anti-sclérose et anti-thrombus (voir page 1 lignes 7-8).

On vient à présent de trouver que les composés β-D-phényl-thioxylosides selon l'invention, structurellement différents des produits connus de l'art antérieur, sont utiles dans le traitement et la prévention des maladies liées aux troubles circulatoires, notamment en tant qu'agents antithrombotiques veineux.

Les composés selon l'invention présentent, de façon inattendue, des propriétés antithrombotiques, qui sont supérieures ou égales à celles des produits décrits dans EP-A-0 290 321 précité, ou qui sont très supérieures à celles des autres produits connus de l'art antérieur, voir à cet effet les résultats des essais comparatifs consignés dans le tableau I ci-après.

Les nouveaux produits selon l'invention sont caractérisés en ce qu'ils sont choisis parmi l'ensemble constitué par les β-D-phényl-thioxylosides de formule :

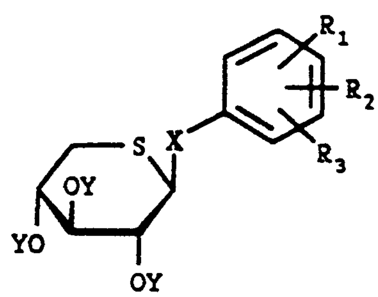

(I)

dans laquelle :

- X représente un atome de soufre ou un atome d'oxygène,
- $R_1$, $R_2$ et $R_3$, identiques ou différents, représentent chacun un atome d'hydrogène, un groupe nitro, un groupe cyano, un groupe -CO-R (où R représente un groupe alkyle en $C_1$-$C_4$ ou un groupe trifluorométhyle), un groupe amino, un groupe acétamido ($NHCOCH_3$), un groupe alkoxy en $C_1$-$C_4$, un groupe trifluorométhyle, un groupe phényle substitué par un ou plusieurs groupes cyano, nitro ou trifluorométhyle, $R_1$ et $R_2$, considérés ensemble, pouvant former avec le groupe phényle auquel ils

sont liés un groupe $\beta$-naphtalényle éventuellement substitué par un ou plusieurs groupes cyano, nitro ou trifluorométhyle ; et,

- Y représente l'atome d'hydrogène ou un groupe acyle aliphatique.

Les fonctions hydroxyles du reste $\beta$-D-thioxylose sont susceptibles d'être acylées, notamment acétylées. La présente invention comprend donc les dérivés de formule I où les fonctions hydroxyles du reste $\beta$-D-thioxylose sont acylées, notamment acétylées.

Parmi les groupes acyles aliphatiques qui conviennent selon l'invention, on peut mentionner ceux qui renferment au total 2 à 5 atomes de carbone, le groupe acyle aliphatique préféré étant $CH_3CO$.

Par groupe alkyle en $C_1$-$C_4$, on entend ici un reste hydrocarboné, ramifié ou linéaire, contenant 1 à 4 atomes de carbone, le groupe alkyle préféré étant le groupe méthyle.

Par groupe alkoxy en $C_1$-$C_4$, on entend ici un groupe alkoxy où le reste hydrocarboné, ramifié ou linéaire, contient 1 à 4 atomes de carbone, le groupe alkoxy préféré étant le groupe méthoxy.

Les composés préférés selon l'invention sont les composés de formule I dans laquelle :

- X représente un atome de soufre ou un atome d'oxygène,
- $R_1$ représente l'atome d'hydrogène,
- l'un au moins des $R_2$ et $R_3$ représente un groupe cyano ou $R_2$ représente l'atome d'hydrogène et $R_3$ représente un groupe 4-acétyle, un groupe 4-acétamido ou un groupe 2-nitro ; et,
- Y représente l'atome d'hydrogène ou un groupe acyle aliphatique en $C_2$-$C_5$.

Les composés de formule I et les composés acylés correspondants peuvent être préparés selon une réaction de glycosylation caractérisée en ce que :

(i) on fait réagir un composé de formule :

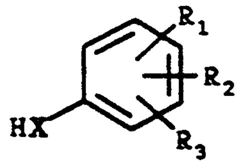

(II)

où X, $R_1$, $R_2$ et $R_3$ sont définis comme ci-dessus,

avec un dérivé du thioxylose choisi parmi l'ensemble comprenant :

(i) les halogénures d'acylthioxylosyle de formule :

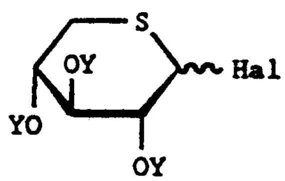

(III)

(ii) les thioxyloses peracylés de formule :

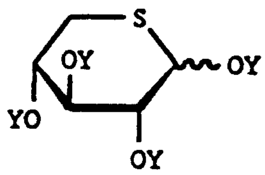

(IV)

(iii) les trichloracétimidates d'acylthioxylosyle de formule :

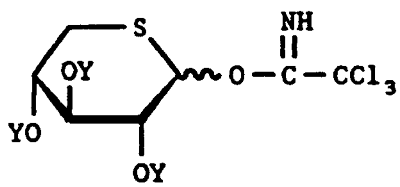

(V)

dans lesquelles Hal représente un atome d'halogène, à savoir Cl ou Br (l'atome de brome étant ici l'atome d'halogène préféré) et Y représente un groupe acyle, notamment un groupe acyle aliphatique comprenant un nombre total d'atomes de carbone de 2 à 5 et de préférence le groupe acétyle,

dans un solvant inerte, à raison de 1 mole de II pour environ 0,6 à 1,2 moles de composé III, IV ou V, en présence d'un accepteur d'acide et/ou d'un acide de Lewis et,

(ii) si nécessaire, on procède à une réaction de désacylation à une température comprise entre O ° C et la température de reflux du milieu réactionnel, dans un alcool inférieur en $C_1$-$C_4$ (de préférence le méthanol), en présence d'un alcoolate métallique (de préférence le méthylate de magnésium ou le méthylate de sodium), pour obtenir un composé de formule I où Y est H.

Les composés III, IV et V peuvent être de configuration $\alpha$ ou $\beta$ ou sous forme d'un mélange anomérique des deux configurations.

Les réactions de glycosylation des phénols et thiophénols II ont été conduites, soit au départ du composé III en présence d'un catalyseur comme les sels ou oxydes d'argent, de mercure ou de zinc, soit au départ du composé V en présence d'un acide de Lewis, notamment l'éthérate de trifluorure de bore ou le chlorure de zinc, soit encore au départ du composé IV en présence d'un acide de Lewis.

Selon un mode préféré de mise en oeuvre de l'invention, on préconise de condenser 1 mole du phénol ou thiophénol II avec environ 1,1 à 1,2 moles d'halogénure d'acylthioxylosyle III dans un solvant inerte choisi parmi les solvants polaires ou apolaires (comme par exemple le diméthylformamide, le tétrahydrofuranne, le dioxanne, l'acétonitrile, le nitrométhane, le benzène, le toluène, les xylènes et leurs mélanges), en présence de cyanure mercurique.

On utilisera avantageusement le bromure de 2,3,4-tri-O-acétyl-5-thio-$\alpha$-D-xylopyranosyle dans un mélange benzène/nitrométhane (1/1) v:v, en présence de 1,1 à 1,3 moles de cyanure mercurique, à une température comprise entre 0 ° C et la température de reflux du milieu réactionnel, de préférence à environ 40-50 ° C, pendant 1 à 4 heures, de préférence pendant environ 2 heures.

Selon un deuxième mode préféré de mise en oeuvre de l'invention, on préconise de condenser 1 mole du phénol ou thiophénol II avec environ 1,1 à 1,2 moles d'halogénure d'acylthioxylosyle III dans un solvant inerte (comme par exemple le dichlorométhane ou l'acétonitrile) en présence d'imidazolate d'argent et de chlorure de zinc.

On utilisera avantageusement le bromure de 2,3,4-tri-O-acétyl-5-thio-D-xylopyranosyle dans du dichlorométhane ou un mélange dichlorométhane/acétonitrile en présence de 1,5 à 1,7 moles d'imidazolate d'argent et de 2 à 2,2 moles de chlorure de zinc, à une température comprise entre 0 ° C et la température de reflux du milieu réactionnel, de préférence à environ 40-60 ° C, pendant 24 à 48 heures.

Selon un troisième mode préféré de mise en oeuvre de l'invention, on préconise de condenser 1 mole du phénol ou thiophénol II avec environ 0,6 à 1 mole d'halogénure d'acylthioxylosyle III dans un solvant inerte (comme par exemple le toluène et/ou l'acétonitrile) en présence d'oxyde de zinc.

On utilisera avantageusement le bromure de 2,3,4-tri-O-acétyl-5-thio-D-xylopyranosyle dans un mélange toluène/acétonitrile, en présence de 0,5 à 1,2 moles d'oxyde de zinc, à une température comprise entre la température ambiante et la température de reflux du milieu réactionnel, de préférence à environ 40-60 ° C, pendant 18 à 48 heures.

Selon un quatrième mode préféré de mise en oeuvre de l'invention, on préconise de condenser 1 mole du phénol ou thiophénol II, avec environ 1,1 à 1,3 moles de trichloracétimidate d'acylthioxylosyle dans un solvant inerte (comme par exemple le dichlorométhane) en présence d'éthérate de trifluorure de bore.

On utilisera avantageusement le trichloracétimidate de 2,3,4-tri-O-acétyl-5-thio-$\alpha$-D-xylopyranosyle dans du dichlorométhane, en présence de 0,1 à 0,4 mole d'éthérate de trifluorure de bore en solution dans du dichlorométhane ou du chlorure de zinc, à une température comprise entre -40 ° C et la température ambiante (15-25 ° C), de préférence à environ -20 ° C à 0 ° C, pendant 1 à 5 heures.

La réaction de glycosylation conduit dans tous les cas à un mélange des isomères de configuration $\alpha$ et $\beta$ en proportions variables.

L'isomère de configuration $\beta$ est isolé selon les méthodes connues de l'homme de l'art, comme par exemple la cristallisation fractionnée ou la chromatographie, notamment la "flash chromatography" [i.e. chromatographie sur colonne de silice et sous pression selon la technique décrite par W.C. STILL et al. dans J. Org. Chem. (1978), 42 (N° 14) 2923].

Les dérivés obtenus sont soumis, le cas échéant, à une désacylation, plus particulièrement à une désacétylation, qui est réalisée à une température comprise entre O°C et la température de reflux du milieu réactionnel dans un alcool inférieur en $C_1$-$C_4$, en présence de l'alcoolate métallique correspondant. De préférence, on choisira le méthanol comme alcool inférieur et le méthanolate de sodium ou de magnésium comme alcoolate métallique.

La réaction de désacylation peut éventuellement être conduite après glycosylation sans isoler le composé acylé intermédiaire formé.

On peut également réaliser la réaction de désacylation par voie enzymatique, par exemple par action de l'estérase de foie de porc.

Les halogénures d'acylthioxylosyle de formule III de configuration $\beta$ et où Y représente un groupe acyle aliphatique renfermant 2 à 5 atomes de carbone sont des composés nouveaux, leurs homologues de configuration $\alpha$ sont connus de EP-A-0 290 321.

Les trichloracétimidates d'acylthioxylosyle de formule V où Y représente un groupe acyle aliphatique renfermant 2 à 5 atomes de carbone sont des composés nouveaux.

Pour accéder aux thiophénols intermédiaires de formule II où X = S on préconise :

(i) de condenser, en milieu basique fort, le chlorure de diméthyl-amino-thiocarbamoyle, de formule :

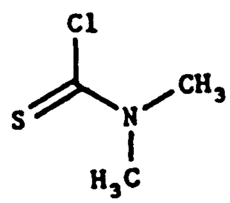

(VI)

sur un phénol de formule :

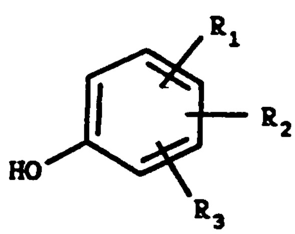

(IIa)

où $R_1$, $R_2$ et $R_3$ ont les significations indiquées ci-dessus, pour obtenir un composé de formule :

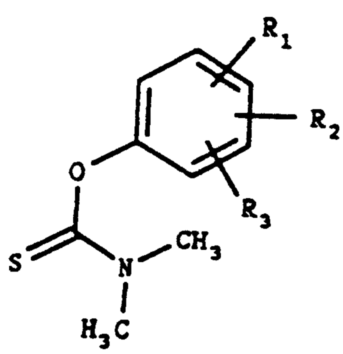

(VII)

où $R_1$, $R_2$ et $R_3$ ont les significations indiquées ci-dessus,

(ii) soumettre le composé de formule VII ainsi obtenu à un réarrangement de Newmann (J. Org. Chem. (1966) 31, p 3980), par chauffage, pour obtenir un composé de formule :

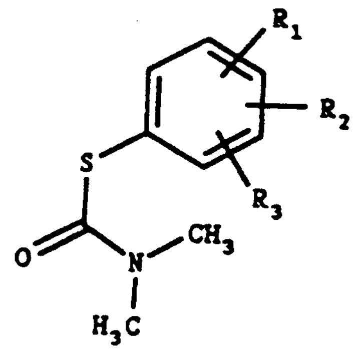

(VIII)

où $R_1$, $R_2$ et $R_3$ ont les significations indiquées ci-dessus,

(iii) traiter le composé de formule VIII ainsi obtenu par un alcoolate métallique, de préférence le méthanolate de sodium ou de magnésium, dans un alcool inférieur en $C_1$-$C_4$, de préférence le méthanol, le diméthylformamide ou le dioxanne pour obtenir un thiophénol de formule II où X = S.

On peut également accéder aux thiophénols intermédiaires par substitution nucléophile d'un halogénobenzène approprié selon la méthode décrite par L. TESTAFERRI dans Tetrahedron Letters Vol. 21 p. 3099-3100 (1980) ou selon la méthode décrite par Paolo BATTISTONI dans Gazzetta Chimica Italiana, 110 p. 301 (1980).

Les thiophénols suivants sont des composés nouveaux :
6-mercapto-2-naphtalènecarbonitrile, et
3,5-dicyano-2-mercapto-benzonitrile.

Les diméthylthiocarbamates suivants sont des composés nouveaux :
diméthylthiocarbamate de O-2-(6-cyanonaphtalényle),
diméthylthiocarbamate de O-3,5-bis(trifluorométhyl)phényle,
diméthylthiocarbamate de O-2,4,6-tricyanophényle,
diméthylthiocarbamate de S-2-(6-cyanonaphtalényle),
diméthylthiocarbamate de S-3,5-bis(trifluorométhyl)phényle, et
diméthylthiocarbamate de S-2,4,6-tricyanophényle.

Selon l'invention, on propose une composition thérapeutique caractérisée en ce qu'elle renferme, en association avec un excipient physiologiquement acceptable, au moins un composé choisi parmi l'ensemble constitué par les produits de formule I. Bien entendu, dans une telle composition l'ingrédient actif intervient en quantité thérapeutiquement efficace.

Les composés de formule I sont utiles en thérapeutique en tant qu'agents antithrombotiques. Ils sont notamment utiles dans la prévention et le traitement des troubles de la circulation veineuse.

Selon l'invention, on préconise l'utilisation d'une substance appartenant à l'ensemble des composés de formule I pour l'obtention d'un médicament antithrombotique destiné à une utilisation thérapeutique vis-à-vis des troubles de la circulation veineuse.

D'autres caractéristiques et avantages de l'invention seront mieux compris à la lecture qui va suivre, d'exemples de préparation, nullement limitatifs, donnés à titre d'illustration, et de résultats d'essais pharmacologiques.

Dans les exemples de préparation qui suivent, les composés ont été nommés en précisant la configuration $\alpha$ ou $\beta$ quand ladite configuration a été déterminée.

Lorsque la configuration n'est pas indiquée, cela signifie que le produit correspondant est un mélange anomérique des configurations $\alpha$ et $\beta$ dans des proportions qui n'ont pas été déterminées.

**PREPARATION I**

**Obtention du 4-cyanophényl 2,3,4-tri-O-acétyl-1,5-dithio-$\beta$-D-xylopyranoside (exemple 1a)**

Sous atmosphère inerte, un mélange de 70 ml de benzène anhydre, de 70 ml de nitrométhane et de 15 g de tamis moléculaire 0,4 nm (commercialisé par la société E. MERCK) est agité à température ambiante (15-25 °C) pendant 0,25 h puis on ajoute 12 g ($47.10^{-3}$ mole) de $Hg(CN)_2$. Après avoir agité le mélange résultant 10 minutes à température ambiante, on ajoute 16,9 g ($47.10^{-3}$ mole) de bromure de 2,3,4-tri-O-acétyl-5-thio-$\alpha$-D-xylopyranosyle puis 6 g ($43.10^{-3}$ mole) de 4-mercapto-benzonitrile par petites fractions. L'addition terminée, le mélange réactionnel est chauffé à 40-50 °C pendant 8 heures puis filtré sur Célite[R] -

(i.e. silice diatomée pour filtration). Le résidu est lavé plusieurs fois avec de l'acétate d'éthyle. La phase organique que l'on recueille est lavée successivement avec une solution aqueuse d'acide chlorhydrique 1N, une solution aqueuse de soude 1N, une solution saturée de chlorure de sodium puis à l'eau jusqu'à pH neutre ; on sèche sur sulfate de magnésium, filtre et évapore le solvant. Le produit brut obtenu est recristallisé dans un mélange acétate d'éthyle/éther de pétrole. On obtient 8,65 g (rendement : 49 %) du produit de configuration $\beta$.
F = 155 °C
$[\alpha]_D^{20°C}$ = + 37 ° (c = 0,5 ; $CHCl_3$)

**PREPARATION II**

**Obtention du 4-cyanophényl 2,3,4-tri-O-acétyl-1,5-dithio-$\beta$-D-xylopyranoside (exemple 1a)**

Une suspension de 625 mg ($1{,}76.10^{-3}$ mole) de bromure de 2,3,4-tri-O-acétyl-5-thio-D-xylopyranosyle, de 200 mg ($1{,}48.10^{-3}$ mole) de 4-mercapto-benzonitrile et de tamis moléculaire 400 pm dans 10 ml d'acétonitrile, est maintenu sous agitation, en présence de 605 mg ($4{,}4.10^{-3}$ mole) de chlorure de zinc et de 310 mg ($1{,}8.10^{-3}$ mole) d'imidazolate d'argent, à l'abri de la lumière, sous atmosphère inerte. Après chauffage à 50 °C pendant 3 h, on filtre le mélange réactionnel sur Célite® dans l'acétate d'éthyle. Le filtrat est lavé au moyen d'une solution d'acide chlorhydrique 1N, d'eau, d'une solution de soude 1N, d'eau et enfin d'une solution saturée de chlorure de sodium, séché sur sulfate de magnésium et évaporé sous pression réduite. Après purification par chromatographie sur gel de silice en éluant au moyen d'un mélange hexane/acétate d'éthyle 3/1 (v/v) et précipitation dans l'éther, on obtient 100 mg (rendement : 17 %) du produit attendu.
F = 155 °C

**PREPARATION III**

**Obtention du 4-cyanophényl 2,3,4-tri-O-acétyl-1,5-dithio-$\beta$-D-xylopyranoside (exemple 1a)**

Une suspension de 192 mg ($0{,}44.10^{-3}$ mole) de trichloracétimidate de 2,3,4-tri-O-acétyl-5-thio-$\alpha$-D-xylopyranosyle, de 71 mg ($0{,}52.10^{-3}$ mole) de 4-mercapto-benzonitrile, de 20 mg ($0{,}15.10^{-3}$ mole) de chlorure de zinc, de tamis 400 pm dans 2 ml d'acétonitrile est maintenue sous agitation pendant 4 h sous atmosphère inerte. Le mélange réactionnel est ensuite filtré sur Célite® dans l'acétate d'éthyle puis lavé au moyen d'une solution de soude 1N, d'eau et enfin d'une solution saturée de chlorure de sodium, séché sur sulfate de magnésium et évaporé sous pression réduite. Après précipitation dans l'éther, on obtient 42 mg (rendement : 23 %) du produit attendu.
F = 155 °C

**PREPARATION IV**

**Obtention du 4-cyanophényl 2,3,4-tri-O-acétyl-1,5-dithio-$\beta$-D-xylopyranoside (exemple 1a)**

Une suspension de 16,9 g ($47.10^{-3}$ mole) de bromure de 2,3,4-tri-O-acétyl-5-thio-$\alpha$-D-xylopyranosyle, de 6 g ($43.10^{-3}$ mole) de 4-mercaptobenzonitrile, de 3,5 g ($43.10^{-3}$ mole) d'oxyde de zinc (ZnO) dans 120 ml de toluène anhydre et 120 ml d'acétonitrile est maintenue sous agitation sous atmosphère inerte en présence de tamis moléculaire (1mm) pendant 18 heures à 50 °C. Après filtration du milieu réactionnel sur Célite® dans l'acétate d'éthyle, la phase organique obtenue est lavée au moyen d'une solution d'HCl 1N deux fois, d'une solution de soude 1N et enfin à l'eau, séchée sur sulfate de magnésium et évaporée sous pression réduite. Après précipitation dans l'éther, on obtient 11,30 g (rendement : 64 %) du produit attendu.
F = 155 °C

**PREPARATION V**

**Obtention du 4-cyanophényl 1,5-dithio-$\beta$-D-xylopyranoside (exemple 1)**

Sous atmosphère d'azote, 8,5 g (21.10-3 mole) de 4-cyanophényl 2,3,4-tri-O-acétyl-1,5-dithio-$\beta$-D-xylopyranoside (exemple 1a) sont mis en suspension dans 100 ml de méthanol puis on ajoute 2 ml de méthylate de sodium (8 % de Na p/v dans le méthanol). Le milieu réactionnel est agité à la température

ambiante jusqu'à dissolution complète du produit de départ (2 heures), puis neutralisé par addition de résine Amberlite$^R$ IR 120H$^+$. Le méthanol est évaporé sous pression réduite ; le produit brut obtenu est recristallisé dans un mélange éthanol/eau 65/25 (v/v). On obtient 5,3 g (rendement : 89,7 %) du produit attendu.
F = 175 °C
$[\alpha]_D^{20°C}$ = + 35,8 ° (c = 0,5 ; $CH_3OH$)

**PREPARATION VI**

**Obtention du 4-nitrophényl 2,3,4-tri-O-acétyl-1,5-dithio-β-D-xylopyranoside (exemple 2a)**

Selon le mode opératoire décrit dans la préparation I, au départ de 6 g ($38.10^{-3}$ mole) de 4-nitrobenzène thiol, de 10,7 g ($42.10^{-3}$ mole) de cyanure mercurique $Hg(CN)_2$ et de 15,1 g ($42.10^{-3}$ mole) de bromure de 2,3,4-tri-O-acétyl-5-thio-α-D-xylopyranosyle, on obtient 10,8 g (rendement : 66 %) du produit attendu.
F = 182 °C
$[\alpha]_D^{20°C}$ = + 50,8 ° (c = 0,64 ; $CHCl_3$)

**PREPARATION VII**

**Obtention du 4-nitrophényl 2,3,4-tri-O-acétyl-1,5-dithio-β-D-xylopyranoside (exemple 2a)**

Selon le mode opératoire décrit dans la préparation IV, au départ de 6 g ($38.10^{-3}$ mole) de 4-nitrobenzenethiol, de 15,1 g ($42.10^{-3}$ mole) de bromure de 2,3,4-tri-O-acétyl-5-thio-α-D-xylopyranosyle et de 3,2 g ($39.10^{-3}$ mole) d'oxyde de zinc (ZnO), on obtient après précipitation dans l'éther 13 g (rendement : 79 %) du produit attendu.
F = 182 °C

**PREPARATION VIII**

**Obtention du 4-nitrophényl 1,5-dithio-β-D-xylopyranoside (exemple 2)**

Selon le mode opératoire décrit dans la préparation V, au départ de 10,3 g ($24.10^{-3}$ mole) de 4-nitrophényl 2,3,4-tri-O-acétyl-1,5-dithio-β-D-xylopyranoside (exemple 2a), on obtient, après recristallisation dans un mélange éthanol/eau 1/1 (v/v), une quantité de 5,4 g (rendement : 74 %) du produit attendu.
F = 168 °C
$[\alpha]_D^{20°C}$ = + 54 ° (c = 0,64 ; $CH_3OH$)

**PREPARATION IX**

**Obtention du 2-naphtalényl 2,3,4-tri-O-acétyl-1,5-dithio-β-D-xylopyranoside (exemple 3a)**

Selon le mode opératoire décrit dans la préparation I, au départ de 6,8 g ($42,4.10^{-3}$ mole) de 2-naphtalènethiol, de 10,8 g ($42,4.10^{-3}$ mole) de cyanure mercurique $Hg(CN)_2$ et de 12 g ($33,2.10^{-3}$ mole) de bromure de 2,3,4-tri-O-acétyl-5-thio-α-D-xylopyranosyle, on obtient 5,84 g (rendement : 40 %) du produit attendu.
F = 151 °C
$[\alpha]_D^{20°C}$ = - 41,5 ° (c = 1,6 ; $CHCl_3$)

**PREPARATION X**

**Obtention du 2-naphtalényl 1,5-dithio-β-D-xylopyranoside (exemple 3)**

Selon le mode opératoire décrit dans la préparation V, au départ de 5,8 g ($13.10^{-3}$ mole) de 2-naphtalényl 2,3,4-tri-O-acétyl-1,5-dithio-α-D-xylopyranoside (exemple 3a), on obtient, après recristallisation dans un mélange éthanol/eau 4/1 (v/v), une quantité de 3,45 g (rendement : 86 %) du produit attendu.
F = 163-164 °C
$[\alpha]_D^{20°C}$ = + 31,1 ° (c = 0,9 ; $CH_3OH$)

## PREPARATION XI

### Obtention du diméthylthiocarbamate de O-4-trifluorométhylphényle

A une solution de 3,63 g ($65.10^{-3}$ mole) de potasse dans 100 ml d'eau et 100 ml d'acétone on additionne 10 g ($62.10^{-3}$ mole) de 4-trifluorométhylphénol. Le mélange obtenu est agité 45 minutes à température ambiante puis refroidi à O°C avant addition de 8,77 g ($71.10^{-3}$ mole) de chlorure de diméthylthiocarbamoyle. Le milieu réactionnel ainsi obtenu est ensuite agité pendant 4 heures à température ambiante, puis hydrolysé. Le produit attendu est extrait à l'acétate d'éthyle. La phase organique ainsi obtenue est lavée au moyen d'une solution aqueuse de soude 1N, puis d'une solution aqueuse d'acide chlorhydrique 1N et enfin à l'eau, puis séchée et évaporée sous pression réduite. On obtient 17 g (rendement quantitatif) du produit attendu.

## PREPARATION XII

### Obtention du diméthylthiocarbamate de S-4-trifluorométhylphényle

Sous atmosphère d'azote 17 g ($68.10^{-3}$ mole) de diméthylthiocarbamate de O-4-trifluorométhylphényle sont portés à 220°C pendant 5 heures. Après purification par "flash chromatography" en éluant avec un mélange toluène/acétate d'éthyle 8/1 (v/v), on obtient 13 g (rendement : 80 %) du produit attendu.

## PREPARATION XIII

### Obtention du 4-trifluorométhylbenzènethiol

Sous atmosphère d'azote, 12 g ($48.10^{-3}$) mole) de diméthylthiocarbamate de S-4-trifluorométhylphényle, sont dissous dans 125 ml de diméthylformamide. La solution obtenue est refroidie à 0°C puis 25 ml de méthylate de sodium en solution à 18 % dans le méthanol sont additionnés. Après 1,5 heure d'agitation, le milieu réactionnel est hydrolysé sur un mélange acide chlorhydrique 1N/glace puis extrait à l'acétate d'éthyle. La phase organique obtenue est lavée à l'eau, séchée sur sulfate de magnésium puis évaporée sous pression réduite. On obtient, après purification par "flash chromatography" en éluant avec un mélange hexane/acétate d'éthyle 8/1 (v/v), une quantité de 6,2 g (rendement : 67 %) du produit attendu.

## PREPARATION XIV

### Obtention du 4-trifluorométhylphényl 2,3,4-tri-O-acétyl-1,5-dithio-$\beta$-D-xylopyranoside (exemple 4a)

Selon le mode opératoire décrit dans la préparation I, au départ de 5,58 g ($32.10^{-3}$ mole) de 4-trifluorométhyl benzènethiol, de 8,87 g ($35.10^{-3}$ mole) de cyanure mercurique $Hg(CN)_2$ et de 12,3 g ($35.10^{-3}$ mole) de bromure de 2,3,4-tri-O-acétyl-5-thio-$\alpha$-D-xylopyranosyle,on obtient 6,2 g (rendement : 40 %) du produit attendu.
F = 160°C
$[\alpha]_D^{20°C}$ = + 16° (c = 0,5 ; $CHCl_3$)

## PREPARATION XV

### Obtention du 4-trifluorométhylphényl 2,3,4-tri-O-acétyl-1,5-dithio-$\beta$-D-xylopyranoside (exemple 4a)

Selon le mode opératoire décrit dans la préparation IV, au départ de 5,6 g ($32.10^{-3}$ mole) de 4-trifluorométhylbenzenethiol, de 12,3 g ($35.10^{-3}$ mole) de bromure de 2,3,4-tri-O-acétyl-5-thio-$\alpha$-D-xylopyranosyle et de 2,55 g ($32.10^{-3}$ mole) d'oxyde de zinc (ZnO), on obtient après précipitation dans l'éther 7,4 g (rendement : 48 %) du produit attendu.
F = 160°C

## PREPARATION XVI

### Obtention du 4-trifluorométhylphényl 1,5-dithio-β-D-xylopyranoside (exemple 4)

Selon le mode opératoire décrit dans la préparation V, au départ de 6,2 g ($14.10^{-3}$ mole) de 4-trifluorométhylphényl 2,3,4-tri-O-acétyl-1,5-dithio-β-D-xylopyranoside (exemple 4a), on obtient, après purification par "flash chromatography" en éluant avec un mélange $CHCl_3/CH_3OH$ 4/1 (v/v), une quantité de 2,05 g (rendement : 45 %) du produit attendu.
F = 128-130 °C
$[\alpha]_D^{23°C}$ = + 10 ° (c = 0,5 : $CH_3OH$)

## PREPARATION XVII

### Obtention du diméthylthiocarbamate de O-3-cyanophényle

Selon le mode opératoire décrit dans la préparation XI, au départ de 15 g ($126.10^{-3}$ mole) de 3-hydroxybenzonitrile, de 17,9 g ($145.10^{-3}$ mole) de chlorure de diméthylthiocarbamoyle et de 7,4 g ($132.10^{-3}$ mole) de potasse, on obtient 29 g (rendement quantitatif) du produit attendu.
F = 108 °C

## PREPARATION XVIII

### Obtention du diméthylthiocarbamate de S-3-cyanophényle

Selon le mode opératoire décrit dans la préparation XII, au départ de 29 g ($141.10^{-3}$ mole) de diméthylthiocarbamate de O-3-cyanophényle, on obtient 19 g (rendement : 65,5 %) du produit attendu.
F = 104 °C

## PREPARATION XIX

### Obtention du 3-mercaptobenzonitrile

Selon le mode opératoire décrit dans la préparation XIII, au départ de 19 g ($92.10^{-3}$ mole) de diméthylthiocarbamate de O-3-cyanophényle, on obtient 10,3 g (rendement : 83,1 %) du produit attendu fondant à 91-95 °C.

## PREPARATION XX

### Obtention du 3-cyanophényl 2,3,4-tri-O-acétyl-1,5-dithio-β-D-xylopyranoside (exemple 5a)

Selon le mode opératoire décrit dans la préparation I, au départ de 9,3 g ($67.10^{-3}$ mole) de 3-mercapto benzonitrile, de 18 g ($73.10^{-3}$ mole) de cyanure mercurique $Hg(CN)_2$ et de 26,14 g ($73.10^{-3}$ mole) de bromure de 2,3,4-tri-O-acétyl-5-thio-α-D-xylopyranosyle,on obtient 8,55 g du produit attendu.
F = 133 °C
$[\alpha]_D^{20°C}$ = + 0,9 ° (c = 0,44 ; $CHCl_3$)

## PREPARATION XXI

### Obtention du 3-cyanophényl 2,3,4-tri-O-acétyl-1,5-dithio-β-D-xylopyranoside (exemple 5a)

Selon le mode opératoire décrit dans la préparation IV, au départ de 10,3 g ($74,1.10^{-3}$ mole) de 3-mercaptobenzonitrile, de 28,94 g ($81,5.10^{-3}$ mole) de bromure de 2,3,4-tri-O-acétyl-5-thio-α-D-xylopyranosyle et de 6,05 g ($74,3.10^{-3}$ mole) d'oxyde de zinc (ZnO), on obtient 9,6 g (rendement : 31 %) du produit attendu.
F = 133 °C

**PREPARATION XXII**

**Obtention du 3-cyanophényl 1,5-dithio-β-D-xylopyranoside (exemple 5)**

Selon le mode opératoire décrit dans la préparation V, au départ de 8,50 g ($20.10^{-3}$ mole) de 3-cyanophényl 2,3,4-tri-O-acétyl-1,5-dithio-β-D-xylopyranoside, on obtient après recristallisation dans le méthanol, 3,10 g (rendement : 54,3 %) du produit attendu.
F = 194-195 °C
$[\alpha]_D^{20°C}$ = - 5,4 ° (c = 0,48 ; $CH_3OH$)

**PREPARATION XXIII**

**Obtention du diméthylthiocarbamate de O-2-cyanophényle**

Selon le mode opératoire décrit dans la préparation XVI, au départ de 15 g ($126.10^{-3}$ mole) de 2-hydroxybenzonitrile, de 17,9 g ($145.10^{-3}$ mole) de chlorure de diméthylthiocarbamoyle et de 7,4 g ($126.10^{-3}$ mole) de potasse, on obtient 24,1 g (rendement : 94 %) du produit attendu.
F = 112 °C

**PREPARATION XXIV**

**Obtention du diméthylthiocarbamate de S-2-cyanophényle**

Selon le mode opératoire décrit dans la préparation XII, au départ de 28 g ($136.10^{-3}$ mole) de diméthylthiocarbamate de O-2-cyanophényle, on obtient 20 g (rendement : 71,4 %) du produit attendu.
F = 70 °C

**PREPARATION XXV**

**Obtention du 2-mercaptobenzonitrile**

Selon le mode opératoire décrit dans la préparation XIII, au départ de 20 g ($97.10^{-3}$ mole) de diméthylthiocarbamate de S-2-cyanophényle, on obtient 10,9 g (rendement : 83,2 %) du produit attendu sous forme d'huile.
$n_D$ = 1,496

**PREPARATION XXVI**

**Obtention du 2-cyanophényl 2,3,4-tri-O-acétyl-1,5-dithio-β-D-xylopyranoside (exemple 6a)**

Selon le mode opératoire décrit dans la préparation I, au départ de 9,70 g ($72.10^{-3}$ mole) de 2-mercapto benzonitrile, de 19,45 g ($77.10^{-3}$ mole) de cyanure mercurique $Hg(CN)_2$ et de 27,4 g ($77.10^{-3}$ mole) de bromure de 2,3,4-tri-O-acétyl-5-thio-α-D-xylopyranosyle on obtient 14,7 g (rendement : 50 %) du produit attendu.
F = 160 °C
$[\alpha]_D^{20°C}$ = - 45,5 ° (c = 0,4 ; $CHCl_3$)

**PREPARATION XXVII**

**Obtention du 2-cyanophényl 2,3,4-tri-O-acétyl-1,5-dithio-β-D-xylopyranoside (exemple 6a)**

Selon le mode opératoire décrit dans la préparation IV, au départ de 10,7 g ($79,2.10^{-3}$ mole) de 2-mercaptobenzonitrile, de 30,2 g ($85.10^{-3}$ mole) de bromure de 2,3,4-tri-O-acétyl-5-thio-α-D-xylopyranosyle et de 6,3 g ($77,4.10^{-3}$ mole) d'oxyde de zinc (ZnO), on obtient après cristallisation dans l'éther 19,3 g (rendement : 60 %) du produit attendu.
F = 160 °C

**PREPARATION XXVIII**

**Obtention du 2-cyanophényl 1,5-dithio-β-D-xylopyranoside (exemple 6)**

Selon le mode opératoire décrit dans la préparation V, au départ de 14,5 g ($35.10^{-3}$ mole) de 2-cyanophényl 2,3,4-tri-O-acétyl-1,5-dithio-β-D-xylopyranoside (exemple 6a), on obtient, après recristallisation dans le méthanol, une quantité de 8,39 g (rendement : 84,6 %) du produit attendu.
F = 118-119 °C
$[\alpha]_D^{20°C}$ = + 12,5 ° (c = 0,52 ; $CH_3OH$)

**PREPARATION XXIX**

**Obtention du 2-nitrobenzènethiol**

15,24 g ($63,4.10^{-3}$ mole) de sulfure de sodium ($Na_2S,9H_2O$) sont ajoutés à une solution de 10 g ($63,4.10^{-3}$ mole) de 2-chloronitrobenzène. La solution obtenue est agitée à température ambiante pendant 12 heures. Le milieu réactionnel est hydrolysé sur un mélange glace/acide chlorhydrique 1N. Le précipité jaune formé est filtré et les eaux mères sont extraites à l'acétate d'éthyle. La phase organique ainsi obtenue est lavée à l'eau jusqu'à pH neutre, séchée sur sulfate de magnésium et évaporée sous pression réduite. On obtient 5,2 g d'huile qui sont ajoutés au 3,8 g de précipité. Ces 9 g de produit résultant sont purifiés par chromatographie sur silice en éluant avec un mélange hexane/acétone 95/5 (v/v). On obtient 6,02 g (rendement : 61 %) du produit attendu.

**PREPARATION XXX**

**Obtention du 2-nitrophényl 2,3,4-tri-O-acétyl-1,5-dithio-β-D-xylopyranoside (exemple 7a)**

Selon le mode opératoire décrit dans la préparation I, au départ de 6 g ($38,7.10^{-3}$ mole) de 2-nitro benzènethiol, de 10,75 g ($42,5.10^{-3}$ mole) de cyanure mercurique $Hg(CN)_2$ et de 15,12 g ($42.10^{-3}$ mole) de bromure de 2,3,4-tri-O-acétyl-5-thio-α-D-xylopyranosyle,on obtient 8 g (rendement : 48 %) du produit attendu.
F= 176 °C
$[\alpha]_D^{20°C}$ = + 15 ° (c = 0,5 ; $CH_2Cl_2/CH_3OH$ 1/1 (v/v))

**PREPARATION XXXI**

**Obtention du 2-nitrophényl 2,3,4-tri-O-acétyl-1,5-dithio-β-D-xylopyranoside (exemple 7a)**

Selon le mode opératoire décrit dans la préparation IV, au départ de 6,1 g ($39,3.10^{-3}$ mole) de 2-nitrobenzenethiol, de 15,40 g ($43,3.10^{-3}$ mole) de bromure de 2,3,4-tri-O-acétyl-5-thio-α-D-xylopyranosyle et de 3,68 g ($45,2.10^{-3}$ mole) d'oxyde de zinc (ZnO), on obtient 9,87 g (rendement : 58 %) du produit attendu.

**PREPARATION XXXII**

**Obtention du 2-nitrophényl 1,5-dithio-β-D-xylopyranoside (exemple 7)**

Selon le mode opératoire décrit dans la préparation V, au départ 8 g ($18,6.10^{-3}$ mole) de 2-nitrophényl 2,3,4-tri-O-acétyl-1,5-dithio-β-D-xylopyranoside (exemple 7a), on obtient, après recristallisation dans le méthanol, une quantité de 4,68 g (rendement : 83,2 %) du produit attendu.
F= 185 °C
$[\alpha]_D^{20°C}$ = +12,4 ° (c = 0,5 ; $CH_2Cl_2/CH_3OH$ 1/1 (v/v))

**PREPARATION XXXIII**

**Obtention du diméthylthiocarbamate de O-2-(6-cyanonaphtalényle)**

A une suspension de 8,05 g ($47.10^{-3}$ mole) de 2-hydroxy-6-naphtalènecarbonitrile dans 50 ml d'acétone et 90 ml d'eau, on additionne 2,85 g ($51.10^{-3}$ mole) de potasse en pastille. On chauffe le mélange

réactionnel à 50 °C pendant trente minutes en agitant vigoureusement. Le mélange est ensuite ramené à 0 °C et on additionne goutte à goutte 6,46 g ($52.10^{-3}$ mole) de chlorure de diméthylthiocarbamoyle dans 80 ml d'acétone. L'addition terminée, on agite pendant trois heures à température ambiante. Le milieu réactionnel est concentré par évaporation sous pression réduite, puis hydrolysé. Après filtration du précipité, on obtient 11,3 g (rendement : 94 %) du produit attendu.
F = 153-154 °C

**PREPARATION XXXIV**

**Obtention du diméthylthiocarbamate de S-2-(6-cyanonaphtalényle)**

Sous atmosphère d'azote et sous agitation, 10 g ($39.10^{-3}$ mole) de diméthylthiocarbamate de O-2-(6-cyanonaphtalényle), sont chauffés à 250 °C pendant six heures. La disparition du produit de départ est contrôlée par chromatographie sur couche mince en éluant avec un mélange acétate d'éthyle/toluène 1/4 (v/v). On obtient 7,6 g (rendement : 76 %) du produit attendu.
F = 166-168 °C

**PREPARATION XXXV**

**Obtention du 6-mercapto-2-naphtalènecarbonitrile**

Sous atmosphère d'azote et sous agitation, 7,15 g ($27{,}9.10^{-3}$ mole) de diméthylthiocarbamate de O-2-(6-cyanonaphtalényle), sont mis en suspension dans 50 ml de dioxanne, puis 16 ml ($55{,}8.10^{-3}$ mole) de méthylate de sodium (solution à 8 % de Na p/v dans le méthanol) sont ajoutés au mélange. Le milieu réactionnel est agité à 21 °C pendant deux heures et on contrôle par chromatographie sur couche mince en éluant avec un mélange acétate d'éthyle/toluène 1/3 (v/v). Le milieu réactionnel est hydrolysé sur un mélange glace/HCl concentré et le précipité formé est filtré. On obtient 5,4 g (rendement : 100 %) du produit attendu.
F = 113-115 °C

**PREPARATION XXXVI**

**Obtention du 2-(6-cyanonaphtalényl) 2,3,4-tri-O-acétyl-1,5-dithio-β-D-xylopyranoside (exemple 8a)**

Selon le mode opératoire décrit dans la préparation I, au départ de 5 g ($27.10^{-3}$ mole) de 6-mercapto-2-naphtalènecarbonitrile, de 7,5 g ($29.10^{-3}$ mole) de cyanure mercurique $Hg(CN)_2$ et de 12 g ($32.10^{-3}$ mole) de bromure de 2,3,4-tri-O-acétyl-5-thio-α-D-xylopyranosyle on obtient 1,6 g (rendement : 12,9 %) du produit attendu.
F = 228-230 °C
$[a]_D^{23°C}$ = + 73,4 ° (c = 0,5 ; $CHCl_3$)

**PREPARATION XXXVII**

**Obtention du 2-(6-cyanonaphtalényl) 2,3,4-tri-O-acétyl-1,5-dithio-β-D-xylopyranoside (exemple 8a)**

Selon le mode opératoire décrit dans la préparation IV, au départ de 5 g ($27.10^{-3}$ mole) de 6-mercapto-2-naphtalènecarbonitrile, de 12 g ($32{,}4.10^{-3}$ mole) de bromure de 2,3,4-tri-O-acétyl-5-thio-α-D-xylopyranosyle et de 2,2 g ($27.10^{-3}$ mole) d'oxyde de zinc (ZnO), on obtient après précipitation dans l'éther 1,5 g du produit attendu.
F = 228-230 °C

**PREPARATION XXXVIII**

**Obtention du 2-(6-cyanonaphtalényl) 1,5-dithio-β-D-xylopyranoside (exemple 8)**

Selon le mode opératoire décrit dans la préparation V, au départ de 1,27 g ($2{,}76.10^{-3}$ mole) de 2-(6-cyanonaphtalényl) 2,3,4-tri-O-acétyl-1,5-dithio-β-D-xylopyranoside (exemple 8a) on obtient, après recristallisation dans un mélange méthanol/chloroforme 1/1 (v/v), une quantité de 0,340 g (rendement : 37 %) du

produit attendu.
F = 226-228 °C
$[\alpha]_D^{24°C}$ = + 45,9 ° (c = 0,3 ; DMSO).

**PREPARATION XXXIX**

**Obtention du phényl 2,3,4-tri-O-acétyl-1,5-dithio-β-D-xylopyranoside(exemple 9a)**

Selon le mode opératoire décrit dans la préparation I, au départ de 4 g ($36,3.10^{-3}$ mole) de benzènethiol, de 14 g ($39,4.10^{-3}$ mole) de bromure de 2,3,4-tri-O-acétyl-5-thio-α-xylopyranosyle et de 10 g ($39.10^{-3}$ mole) de cyanure mercurique ($Hg(CN)_2$), on obtient après cristallisation dans l'éther 7,3 g du produit attendu.
F = 130 °C
$[\alpha]_D^{20°C}$ = + 14,6 ° (c = 0,5 ; $CHCl_3$)

**PREPARATION XL**

**Obtention du phényl 2,3,4-tri-O-acétyl-1,5-dithio-β-D-xylopyranoside(exemple 9a)**

Selon le mode opératoire décrit dans la préparation IV, au départ de 4 g ($36,3.10^{-3}$ mole) de benzenethiol, de 15 g ($42,3.10^{-3}$ mole) de bromure de 2,3,4-tri-O-acétyl-5-thio-α-D-xylopyranosyle et de 3 g ($36,8.10^{-3}$ mole) d'oxyde de zinc (ZnO), on obtient après cristallisation dans l'éther 4,5 g (rendement : 32,29 %) du produit attendu.
F = 130 °C

**PREPARATION XLI**

**Obtention du phényl 1,5-dithio-β-D-xylopyranoside (exemple 9)**

Selon le mode opératoire décrit dans la préparation V, au départ de 6,9 g ($18.10^{-3}$ mole) de phényl 2,3,4-tri-O-acétyl-1,5-dithio-β-D-xylopyranoside (exemple 9a), on obtient, après recristallisation dans un mélange éthanol/eau 50/10 (v/v), une quantité de 3,7 g (rendement : 80 %) du produit attendu.
F = 150-151 °C
$[\alpha]_D^{20°C}$ = - 6 ° (c = 0,5 ; $CH_3OH$)

**PREPARATION XLII**

**Obtention du 3,4,5-triméthoxyphényl 2,3,4-tri-O-acétyl-1,5-dithio-β-D-xylopyranoside (exemple 10a)**

Selon le mode opératoire décrit dans la préparation I, au départ de 11,35 g ($57.10^{-3}$ mole) de 3,4,5-triméthoxybenzènethiol, de 14,32 g ($57.10^{-3}$ mole) de cyanure mercurique $Hg(CN)_2$ et de 22,15 g ($62.10^{-3}$ mole) de bromure de 2,3,4-tri-O-acétyl-5-thio-α-D-xylopyranosyle, on obtient 7,52 g (rendement : 28 %) du produit attendu.
F = 101 °C
$[\alpha]_D^{25°C}$ = - 43 ° (c = 0,2 ; $CH_3OH$)

**PREPARATION XLIII**

**Obtention du 3,4,5-triméthoxyphényl 1,5-dithio-β-D-xylopyranoside (exemple 10)**

Selon le mode opératoire décrit dans la préparation V, au départ de 4,65 g ($9,8.10^{-3}$ mole) de 3,4,5-triméthoxyphényl 2,3,4-tri-O-acétyl-1,5-dithio-β-D-xylopyranoside (exemple 10a), on obtient, après recristallisation dans un mélange méthanol/eau 1/1 (v/v), une quantité de 2,4 g (rendement : 70 %) du produit attendu.
F = 166 °C
$[\alpha]_D^{20°C}$ = - 12 ° (c = 0,2 ; $CH_3OH$)

**PREPARATION XLIV**

**Obtention du 4-acétylphényl 2,3,4-tri-O-acétyl-1,5-dithio-β-D-xylopyranoside (exemple 11a)**

Selon le mode opératoire décrit dans la préparation I, au départ de 1,03 g ($6,7.10^{-3}$ mole) de 4-mercaptoacétophénone, de 1,72 g ($6,8.10^{-3}$ mole) de cyanure mercurique $Hg(CN)_2$ et de 2,65 g ($7,5.10^{-3}$ mole) de bromure de 2,3,4-tri-O-acétyl-5-thio-α-D-xylopyranosyle, on obtient 0,36 g (rendement : 12,5 %) du produit attendu.
F = 122 °C
$[\alpha]_D^{22°C}$ = + 46,5 ° (c = 0,29 $CHCl_3$)

**PREPARATION XLV**

**Obtention du 4-acétylphényl 2,3,4-tri-O-acétyl-1,5-dithio-β-D-xylopyranoside (exemple 11a)**

Selon le mode opératoire décrit dans la préparation IV, au départ de 1,27 g ($8,43.10^{-3}$ mole) de 4-mercaptoacétophénone, de 3,27 g ($9,2.10^{-3}$ mole) de bromure de 2,3,4-tri-0-acétyl-5-thio-α-D-xylopyranosyle et de 0,68 g ($8,35.10^{-3}$ mole) d'oxyde de zinc (ZnO), on obtient 0,42 g (rendement : 11 %) du produit attendu.
F = 122 °C

**PREPARATION XLVI**

**Obtention du 4-acétylphényl 1,5-dithio-β-D-xylopyranoside (exemple 11)**

Selon le mode opératoire décrit dans la préparation V, au départ de 0,34 g (0,8. $10^{-3}$ mole) de 4-acétylphényl 2,3,4-tri-O-acétyl-1,5-dithio-β-D-xylopyranoside (exemple 11a), on obtient après recristallisation dans un mélange méthanol/eau 1/1 (v/v), une quantité de 0,12 g (rendement : 50 %) du produit attendu.
F = 175 °C
$[\alpha]_D^{25°C}$ = + 34 ° (c = 0,2 ; $CH_3OH$)

**PREPARATION XLVII**

**Obtention du 3-nitrophényl 2,3,4-trio-O-acétyl-1,5-dithio-β-D-xylopyranoside (exemple 12a)**

Selon le mode opératoire décrit dans la préparation I, au départ de 10 g ($64,5.10^{-3}$ mole) de 3-nitrobenzènethiol, de 16,29 g ($64,5.10^{-3}$ mole) de cyanure mercurique ($Hg(CN)_2$), de 25,2 g ($70,9.10^{-3}$ mole) de bromure de 2,3,4-tri-O-acétyl-5-thio-α-D-xylopyranosyle, on obtient, après purification par "flash chromatography" en éluant au moyen d'un mélange toluène/acétate d'éthyle 9/1 (v/v) puis cristallisation dans l'éther, une quantité de 7,44 g (rendement : 27 %) du produit attendu.
F = 121 °C
$[\alpha]_D^{20°C}$ = + 1,8 ° (c = 0,5 ; $CH_3OH$)

**PREPARATION XLVIII**

**Obtention du 3-nitrophényl 1,5-dithio-β-D-xylopyranoside (exemple 12)**

Selon le mode opératoire décrit dans la préparation V, au départ de 7,18 g ($16,7.10^{-3}$ mole) de 3-nitrophényl 2,3,4-tri-O-acétyl-1,5-dithio-β-D-xylopyranoside (exemple 12a), on obtient 3,9 g (rendement : 77 %) du produit attendu.
F = 152-154 °C
$[\alpha]_D^{20°C}$ = -3,6 ° (c = 0,5 ; $CH_3OH$)

**PREPARATION IL**

**Obtention du diméthylthiocarbamate de O-2-trifluorométhylphényle**

Selon le mode opératoire décrit dans la préparation XI, au départ de 3,95 g (24,3.10-$^3$ mole) de 2-trifluorométhylphénol, de 1,43 g (25,6.10-$^3$ mole) de potasse et de 3,46 g (28.10-$^3$ mole) de chlorure de diméthylthiocarbamoyle on obtient 5,37 g (rendement : 89 %) d'huile jaune.
$n_D^{24,5°C}$ = 1,528

**PREPARATION L**

**Obtention du diméthylthiocarbate de S-2-trifluorométhylphényle**

Selon le mode opératoire décrit dans la préparation XII, au départ de 5,37 g (21,5.10-$^3$ mole) de diméthylthiocarbamate de O-2-trifluorométhylphényle, on obtient, après purification par "flash chromatography" en éluant avec un mélange toluène/acétate d'éthyle 98/2 (v/v), une quantité de 3,2 g (rendement : 60 %) du produit attendu.
$n_D^{25°C}$ = 1,5182

**PREPARATION LI**

**Obtention du 2-trifluorométhylbenzènethiol**

Selon le mode opératoire décrit dans la préparation XIII, au départ de 2,72 g (10,9.10-$^3$ mole) de diméthylthiocarbamate de S-2-trifluorométhylphényle, on obtient 2 g (rendement quantitatif) du produit attendu.

**PREPARATION LII**

**Obtention du 2-trifluorométhylphényl 2,3,4-tri-O-acétyl-1,5-dithio-β-D-xylopyranoside (exemple 13a)**

Selon le mode opératoire décrit dans la préparation I, au départ de 1,8 g (10.10-$^3$ mole) de 2-trifluorométhyl benzènethiol, de 2,55 g (10.10-$^3$ mole) de cyanure mercurique ($Hg(CN)_2$) et de 3,95 g (11.10-$^3$ mole) de bromure de 2,3,4-tri-O-acétyl-5-thio-α-D-xylopyranosyle,on obtient 1,53 g (rendement : 34 %) du produit attendu.
F = 152 °C
$[\alpha]_D^{20°C}$ = + 64 ° (c = 0,5 ; $CH_3OH$)

**PREPARATION LIII**

**Obtention du 2-trifluorométhylphényl 1,5-dithio-β-D-xylopyranoside (exemple 13)**

Selon le mode opératoire décrit dans la préparation V, au départ de 1,38 g (3.10-$^3$ mole) de 2-trifluorométhylphényl 2,3,4-tri-O-acétyl-1,5-dithio-β-D-xylopyranoside (exemple 13a), on obtient, après recristallisation dans un mélange méthanol/eau 1/1 (v/v), une quantité de 0,75 g (rendement : 75 %) du produit attendu.
F = 114-115 °C
$[\alpha]_D^{20°C}$ = + 34 ° (c = 0,5 ; $CH_3OH$)

**PREPARATION LIV**

**Obtention du 4-(4-iodophényl)benzonitrile**

Un mélange de 15 g (33,2.10-$^3$ mole) de 4,4′-diiodo-1,1′-biphényle de 3,13 g (34,9.10-$^3$ mole) de cyanure mercurique et de 2,75 g (34,9.10-$^3$ mole) de pyridine est chauffé à 200 °C pendant 15 minutes puis 6 ml de diméthylformamide sont ajoutés au mélange. Après refroidissement le milieu réactionnel est hydrolysé par une solution aqueuse d'acide chlorhydrique 1N. Le produit attendu est extrait à l'acétate

d'éthyle. La phase organique obtenue est lavée à l'eau jusqu'à pH neutre, séchée sur sulfate de magnésium et évaporée sous vide. On obtient après purification par "flash chromatography" en éluant avec un mélange chloroforme/toluène 1/1 (v/v), une quantité de 3,28 g (rendement : 33 %) de solide jaune.
F = 162-168 °C

**PREPARATION LV**

**Obtention du 4-(4-mercaptophényl)benzonitrile**

3,25 g ($10,8.10^{-3}$ mole) de 4-(4-iodophényl)benzonitrile sont dissous dans 50 ml d'hexaméthylphosphoramide puis 3,05 g ($43,4.10^{-3}$ mole) de thiométhylate de sodium sont ajoutés à la solution. Le mélange réactionnel est chauffé à 100 °C pendant 1,5 heure puis, après refroidissement, hydrolysé sur un mélange acide chlorhydrique 1N/glace. Le produit attendu est extrait à l'acétate d'éthyle. La phase organique ainsi obtenue est lavée à l'eau jusqu'à pH neutre, séchée sur sulfate de magnésium puis évaporée sous pression réduite. On obtient 2,4 g (rendement quantitatif) de solide jaune pâle.
F = 105-115 °C

**PREPARATION LVI**

**Obtention du 4-(4-cyanophényl)phényl 2,3,4-tri-O-acétyl-1,5-dithio-β-D-xylopyranoside (exemple 14a)**

Selon le mode opératoire décrit dans la préparation I, au départ de 2,3 g ($10,9.10^{-3}$ mole) de 4-(4-mercaptophényl)benzonitrile, de 4,26 g ($11,9.10^{-3}$ mole) de bromure de 2,3,4-tri-O-acétyl-5-thio-α-D-xylopyranosyle et de 2,75 g ($10,9.10^{-3}$ mole) de cyanure mercurique ($Hg(CN)_2$), on obtient, après purification par "flash chromatography" en éluant avec du chlorure de méthylène, une quantité de 0,540 g (rendement : 10 %) du produit attendu.
F = 150 °C
$[\alpha]_D^{20°C}$ = + 10,2 ° (c = 0,5 ; $CHCl_3$)

**PREPARATION LVII**

**Obtention du 4-(4-cyanophényl)phényl 1,5-dithio-β-D-xylopyranoside (exemple 14)**

Selon le mode opératoire décrit dans la préparation V, au départ de 0,340 g ($70,1.10^{-3}$ mole) de 4-(4-cyanophényl)phényl 2,3,4-tri-O-acétyl-1,5-dithio-β-D-xylopyranoside (exemple 14a) et de 17 ml de méthylate de sodium (solution à 8 % de Na p/v dans le méthanol), on obtient après purification par recristallisation dans le méthanol 0,200 g (rendement : 80 %) du produit attendu.
F = 168 °C
$[\alpha]_D^{20°C}$ = + 5,4 ° (c = 0,5 ; $CH_3OH/CH_3Cl$ 1/1 (v/v))

**PREPARATION LVIII**

**Obtention du diméthylthiocarbamate de O-3,5-bis(trifluorométhyl)phényl**

0,975 g ($17,4.10^{-3}$ mole) de potasse en pastilles sont dissous dans 60 ml d'eau, puis 3,81 g ($16,5.10^{-3}$ mole) de 3,5-bis(trifluorométhyl)benzènethiol sont additionnés et le mélange obtenu est agité pendant 20 minutes à température ambiante. Une solution de 2,35 g ($19.10^{-3}$ mole) de chlorure de diméthylthiocarbamoyle dans 60 ml d'acétone est ensuite ajoutée goutte à goutte puis la solution ainsi obtenue est agitée pendant 30 minutes à température ambiante. Le milieu réactionnel est hydrolysé sur un mélange de glace et d'acide chlorhydrique 1N, puis extrait à l'acétate d'éthyle. La phase organique est lavée à l'eau jusqu'à pH neutre, séchée sur sulfate de magnésium, décolorée au noir animal et évaporée sous pression réduite. On obtient 4,81 g du produit attendu (rendement : 92 %) sous forme d'un solide jaune pâle.
F = 71-80 °C

**PREPARATION LIX**

**Obtention du diméthylthiocarbamate de S-3,5-bis(trifluorométhyl)phényle**

4,81 g ($15.10^{-3}$ mole) du diméthylthiocarbamate de O-3,5-bis(trifluorométhyl)phényl sont chauffés à 200-210 °C pendant 2 heures. On obtient 2,81 g (rendement : 58,4%) du produit attendu sous forme d'huile jaune.
$n_D^{25°C}$ = 1,4710

**PREPARATION LX**

**Obtention du 3,5-bis(trifluorométhyl)benzènethiol**

2,25 g ($7,1.10^{-3}$ mole) de diméthylthiocarbamate de S-3,5-bis(trifluorométhyl)phényle, sont dissous dans 2,2 ml de diméthylformamide anhydre. La solution ainsi obtenue est refroidie à 0 °C avant l'addition de 4 ml ($14.10^{-3}$ mole) de méthylate de sodium en solution dans le méthanol, puis agitée à 0 °C pendant 10 minutes. Le milieu réactionnel est ensuite hydrolysé par un mélange glace/eau/HCl 1N puis extrait au chlorure de méthylène. La phase organique obtenue est lavée au moyen d'une solution saturée de chlorure de sodium, séchée sur sulfate de magnésium et évaporée sous pression réduite. On obtient 1,74 g (rendement : 100 %) du produit attendu sous forme d'une huile jaune qui cristallise et dimérise sous l'action de la chaleur.
Point de fusion du dimère = 71 °C

**PREPARATION LXI**

**Obtention du 3,5-bis(trifluorométhyl)phényl 2,3,4-tri-O-acétyl-1,5-dithio-β-D-xylopyranoside (exemple 15a)**

Selon le mode opératoire décrit dans la préparation I, au départ de 1,6 g ($6,5.10^{-3}$ mole) de 3,5-bis-(trifluorométhyl)benzènethiol, de 1,64 g ($6,5.10^{-3}$ mole) de cyanure mercurique $Hg(CN)_2$ et de 2,54 g ($7,5.10^{-3}$ mole) de bromure de 2,3,4-tri-O-acétyl-5-thio-α-D-xylopyranosyle, on obtient 1,2 g (rendement : 35,7 %) du produit attendu.
F = 201 °C
$[\alpha]_D^{20°C}$ = + 6 ° (c = 0,5 ; $CHCl_3$)

**PREPARATION LXII**

**Obtention du 3,5-bis(trifluorométhyl)phényl 1,5-dithio-β-D-xylopyranoside (exemple 15)**

Selon le mode opératoire décrit dans la préparation V, au départ de 1,1 g ($2,1.10^{-3}$ mole) de 3,5-bis-(trifluorométhyl)phényl 2,3,4-tri-O-acétyl-1,5-dithio-β-D-xylopyranoside, on obtient, après recristallisation dans un mélange méthanol/eau 1/1 (v/v), une quantité de 0,600 g (rendement : 72 %) du produit attendu.
F = 157-158 °C
$[\alpha]_D^{20°C}$ = + 3 ° (c = 0,5 ; $CH_3OH$)

**PREPARATION LXIII**

**Obtention du 3-cyano-4-mercaptobenzonitrile**

Sous argon, 3,03 g ($12.10^{-3}$ mole) de 3-cyano-4-iodo benzonitrile et 3,36 g ($48.10^{-3}$ mole) de thiométhylate de sodium sont dissous dans 80 ml d'hexaméthylphosphoramide anhydre puis la solution obtenue est chauffée à 80 °C pendant 45 minutes. Le milieu réactionnel ainsi obtenu est hydrolysé sur un mélange glace/HCl 1N, puis extrait au chlorure de méthylène. La phase organique est lavée à l'eau puis séchée sur sulfate de magnésium et évaporée sous pression réduite. On obtient 1,55 g (rendement : 81 %) du produit attendu.
F = 180 °C

**PREPARATION LXIV**

**Obtention du 2,4-dicyanophényl 2,3,4-tri-O-acétyl-1,5-dithio-β-D-xylopyranoside (exemple 16a)**

Selon le mode opératoire décrit dans la préparation I, au départ de 1,5 g (9.10-$^3$ mole) de 3-cyano-4-mercapto benzonitrile, de 2,78 g (11.10-$^3$ mole) de cyanure mercurique $Hg(CN)_2$ et de 3,66 g (11.10-$^3$ mole) de bromure de 2,3,4-tri-O-acétyl-5-thio-α-D-xylopyranosyle,on obtient 1,65 g (rendement : 40,5 %) du produit attendu.
F = 228 °C
$[\alpha]_D^{20°C}$ = - 14 ° (c = 0,39 ; $CHCl_3$)

**PREPARATION LXV**

**Obtention du 2,4-dicyanophényl 2,3,4-tri-O-acétyl-1,5-dithio-β-D-xylopyranoside (exemple 16a)**

Selon le mode opératoire décrit dans la préparation IV, au départ de 1,6 g (9,73.10-$^3$ mole) de 3-cyano-4-mercaptobenzonitrile, de 4,07 g (11,45.10-$^3$ mole) de bromure de 2,3,4-tri-O-acétyl-5-thio-α-D-xylopyranosyle et de 0,84 g (10.10-$^3$ mole) d'oxyde de zinc (ZnO), on obtient 2,08 g (rendement : 47 %) du produit attendu.
F = 228 °C

**PREPARATION LXVI**

**Obtention du 2,4-dicyanophényl 1,5-dithio-β-D-xylopyranoside (exemple 16)**

Selon le mode opératoire décrit dans la préparation V, mais en opérant à 0 °C,au départ de 1,5 g (34.10-$^3$ mole) de 2,4-dicyanophényl 2,3,4-tri-O-acétyl-1,5-dithio-β-D-xylopyranoside (exemple 16a), on obtient après chromatographie sur silice en éluant au moyen d'un mélange $CH_2Cl_2$ / $CH_3OH$ 8/1 (v/v), une quantité de 0,71 g (rendement : 67 %) du produit attendu.
F = 180-181 °C
$[\alpha]_D^{20°C}$ = + 42,7 ° (c = 0,48 ; $CH_3OH$)

**PREPARATION LXVII**

**Obtention du 3,5-dicyano-2-mercaptobenzonitrile**

Selon le mode opératoire décrit dans la préparation LV, au départ de 6 g (26.10-$^3$ mole) de 2-bromo-3,5-dicyanobenzonitrile et de 6 g (86.10-$^3$ mole) de thiométhylate de sodium, on obtient 6 g (rendement quantitatif) du produit attendu sous forme d'huile.
$n_D^{29,5°C}$ = 1,5012

**PREPARATION LXVIII**

**Obtention du 2,4,6-tricyanophényl 2,3,4-tri-O-acétyl-1,5-dithio-β-D-xylopyranoside (exemple 17a)**

Selon le mode opératoire décrit dans la préparation I, au départ de 4,8 g (258.10-$^3$ mole) de 3,5-dicyano-2-mercaptobenzonitrile (exemple 17a), de 9,71 g (258.10-$^3$ mole), de bromure de 2,3,4-tri-O-acétyl-5-thio-α-D-xylopyranosyle et de 6,57g (8,6.10-$^3$ mole) de cyanure mercurique ($Hg(CN)_2$) on obtient après cristallisation dans l'éther 2 g (rendement : 17 %) du produit attendu.
F = 221 °C
$[\alpha]_D^{20°C}$ = + 84,6 ° (c = 0,325 ; $CHCl_3$)

**PREPARATION LXIX**

**Obtention du 2,4,6-tricyanophényl 1,5-dithio-β-D-xylopyranoside (exemple 17)**

0,600 g (1,30.10-$^3$ mole) de 2,4,5-tricyanophényl 2,3,4-tri-O-acétyl-1,5-dithio-β-D-xylopyranoside est mis en suspension dans 120 ml de tampon 0,1 M $Na_2HPO_4/NaH_2PO_4$ (pH : 7,35) et 3 gouttes de Triton$^R$ X100

(commercialisé par la Société SIGMA). On ajoute ensuite 30 gouttes d'estérase de foie de porc [Sigma type I suspension 3,2 M dans $(NH_4)_2SO_4$] (commercialisé par la Société SIGMA) et le mélange ainsi obtenu est agité à 30-35 °C pendant 12 heures. On ajoute à nouveau 20 gouttes d'estérase de foie de porc et 10 gouttes de Triton$^R$ X100 et après 24 heures d'agitation on ajoute enfin 30 gouttes d'estérase de foie de porc. Le pH est maintenu à 7,4 par addition de soude 1N pendant toute la durée de l'expérience. Après 84 heures d'agitation le mélange réactionnel est refroidi et le produit attendu est extrait à l'acétate d'éthyle. La phase organique ainsi obtenue est lavée à la saumure puis séchée sur sulfate de magnésium et évaporée sous pression réduite. On obtient, après purification par "flash chromatography" en éluant avec un mélange $CHCl_3/CH_3OH$ 98/2 (v/v) puis 95/5 (v/v), une quantité de 100 mg (rendement : 23 %) du produit attendu sous forme de mousse. Il s'agit d'un produit hydraté contenant 1,3 $H_2O$ par molécule.

F = 86-96 °C

$[\alpha]_D^{20°C}$ = 0 ° (c = 0,165 ; $CH_3OH$)

**PREPARATION LXX**

**Obtention du 4-aminophényl 1,5-dithio-β-D-xylopyranoside (exemple 18)**

A une solution de 1,7 g ($5,61.10^{-3}$ mole) de 4-nitrophényl 1,5-dithio-β-D-xylopyranoside dans 150 ml de méthanol on ajoute 170 mg de charbon palladié à 10 %. Le milieu réactionnel est maintenu sous pression d'hydrogène ($3,5.10^5$ Pa), à température ambiante pendant 3 jours. Des additions répétées de 170 mg de charbon palladié à 10 % sont effectuées après 3 heures, 4 heures, 12 heures, 24 heures d'agitation. Le mélange obtenu est filtré, le solvant est évaporé sous pression réduite et le résidu obtenu est purifié par "flash chromatography" en éluant au moyen d'un mélange $CHCl_3/CH_3OH$ 9/1 (v/v) puis recristallisé dans l'eau. On obtient 0,7 g (rendement : 46 %) du produit attendu.

F = 163-166 °C

$[\alpha]_D^{23°C}$ = - 74 ° (c = 0,102 ; DMSO)

**PREPARATION LXXI**

**Obtention du 4-acétamidophényl 2,3,4-tri-O-acétyl-1,5-dithio-β-D-xylopyranoside (exemple 19a)**

Selon le mode opératoire décrit dans la préparation IV, au départ de 4,5 g ($27.10^{-3}$ mole) de N-(4-mercaptophényl)acétamide, de 11,43 g ($32.10^{-3}$ mole) de bromure de 2,3,4-tri-O-acétyl-5-thio-D-xylopyranosyle et de 2,16 g ($27.10^{-3}$ mole) d'oxyde de zinc (ZnO), on obtient après recristallisation dans un mélange toluène/éther isopropylique 3 g (rendement : 25 %) du produit attendu.

F = 168-174 °C

$[\alpha]_D^{23°C}$ = + 8 ° (c = 0,5 ; $CHCl_3$)

**PREPARATION LXXII**

**Obtention du 4-acétamidophényl 1,5-dithio-β-D-xylopyranoside (exemple 19)**

Selon le mode opératoire décrit dans la préparation V, au départ de 1,05 g ($2,38.10^{-3}$ mole) de 4-acétomidophényl 2,3,4-tri-O-acétyl-1,5-dithio-β-D-xylopyranoside (exemple 19a), on obtient après recristallisation dans 70 ml d'eau 0,61 g (rendement : 81 %) du produit attendu.

F = 226-233 °C

$[\alpha]_D^{23°C}$ = - 25,25 ° (c = 0,59 ; DMSO)

**PREPARATION LXXIII**

**Obtention du 4-trifluoroacétylphényl 2,3,4-tri-O-acétyl-1,5-dithio-β-D-xylopyranoside (exemple 20a)**

Selon le mode opératoire décrit dans la préparation I, au départ de 9,03 g ($43,8.10^{-3}$ mole) de 2,2,2-trifluoro-1-(4-mercaptophényl) éthanone, de 11,5 g ($45,5.10^{-3}$ mole) de cyanure mercurique et de 17,1 g ($48,2.\ 10^{-3}$ mole) de 2,3,4-tri-O-acétyl-1-bromo-5-thio-D-xylopyranoside, on obtient après purification par "flash chromatography" en éluant au moyen d'un mélange toluène/éther 8/2 (v/v) et recristallisation dans l'éther 4,79 g (rendement : 22 %) du produit attendu.

F = 143-148 °C

$[\alpha]_D^{24°C}$ = + 59,3° (c = 0,28 ; $CHCl_3$)

**PREPARATION LXXIV**

**Obtention du 4-trifluoroacétylphényl 1,5-dithio-β-D-xylopyranoside (exemple 20)**

Selon le mode opératoire décrit dans la préparation V, au départ de 3,65 g (7,6.$10^{-3}$ mole) de 4-trifluoroacétylphényl 2,3,4-tri-O-acétyl-1,5-dithio-β-D-xylopyranoside (exemple 20a), on obtient, après purification par recristallisation dans un mélange toluène/alcool n-propylique/hexane, 1,4 g (rendement : 52 %) du produit attendu.
F = 133-134°C
$[\alpha]_D^{22°C}$ = + 15 (c = 0,31 ; $CH_3OH$)

**PREPARATION LXXV**

**Obtention du 3-aminophényl 1,5-dithio-β-D-xylopyranoside (exemple 21)**

Selon le mode opératoire décrit dans la préparation LXX, au départ de 2,9 g (9,6.$10^{-3}$ mole) de 3-nitrophényl 1,5-dithio-β-D-xylopyranoside, après purification par "flash chromatography" en éluant au moyen d'un mélange $CHCl_3/CH_3OH$ (quotient d'élution de 95/5 à 92/8 (v/v)) et précipitation dans un mélange $CH_3OH$/éther, on obtient 1,2 g (rendement : 46 %) du produit attendu.
F = 128-132°C
$[\alpha]_D^{25°C}$ = + 3,5° (c = 0,31 ; $CH_3OH$)

**PREPARATION LXXVI**

**Obtention du 4-cyanophényl 2,3,4-tri-O-acétyl-5-thio-β-D-xylopyranoside (exemple 22a)**

Une suspension de 6,5 g (12,3.$10^{-3}$ mole) de bromure de 2,3,4-tri-O-acétyl-5-thio-α-D-xylopyranosyle, de 6 g (50,4.$10^{-3}$ mole) de 4-hydroxybenzonitrile, de 6,9 g (50,5.$10^{-3}$ mole) de chlorure de zinc et de 4,4 g (25,1.$10^{-3}$ mole) d'imidazolate d'argent dans 200 ml de dichlorométhane anhydre est maintenue à 40°C sous agitation, sous atmosphère inerte, à l'abri de la lumière et en présence de tamis moléculaire (400 pm). Après 7 h à cette température, on ajoute 6,9 g (50,5.$10^{-3}$ mole) de chlorure de zinc, 4,4 g (25,1.$10^{-3}$mole) d'imidazolate d'argent et 6,5 g (18,3.$10^{-3}$ mole) de bromure de 2,3,4-tri-O-acétyl-5-thio-α-D-xylopyranosyle. On laisse dans ces conditions pendant une nuit et on additionne alors 6,5 g (12,3.$10^{-3}$) mole) de bromure de 2,3,4-tri-O-acétyl-5-thio-α-D-xylopyranosyle. Après 24 h, le mélange réactionnel est filtré sur Célite®, lavé au moyen d'une solution aqueuse d acide chlorhydrique 1N, puis à l'eau et séché sur sulfate de magnésium. Après évaporation sous pression réduite, le résidu est purifié par chromatographie sur gel de silice en éluant avec un mélange hexane/acétate d'éthyle, 3/1 (v/v). Par cristallisation dans l'éthanol, on obtient 8,1 g (rendement : 41 %) du produit attendu.
F = 145-148°C
$[\alpha]_D^{21°C}$ = - 29° (c = 0,47 ; $CHCl_3$)

**PREPARATION LXXVII**

**Obtention du bromure de 2,3,4-tri-O-acétyl-5-thio-β-D-xylopyranosyle**

A une solution de 2,10 g (6,3.$10^{-3}$ mole) de 1,2,3,4-tétra-O-acétyl-5-thio-D-xylopyranose dans 10 $cm^3$ de dichloroéthane, on ajoute, à 10°C, 3,50 ml d'une solution d'acide bromhydrique à 30 % dans l'acide acétique glacial. Après 2 à 3 h, le milieu réactionnel est hydrolysé, lavé au moyen d'une solution de bicarbonate de sodium, séché sur sulfate de sodium ($Na_2SO_4$) et le solvant est évaporé à sec sous pression réduite. Après précipitation dans l'éther, on obtient 0,87 g (rendement : 39 %) du produit attendu.
F = 175°C
$[\alpha]_D^{21°C}$ = - 67° (c = 0,56 ; $CHCl_3$)

**PREPARATION LXXVIII**

**Obtention du 4-cyanophényl 2,3,4-tri-O-acétyl-5-thio-β-D-xylopyranoside (exemple 22a)**

Une suspension de 0,5 g (1,4.$10^{-3}$) mole) de bromure de 2,3,4-tri-O-acétyl-5-thio-D-xylopyranosyle, de 0,25 g (2,1.$10^{-3}$ mole) de 4-hydroxybenzonitrile, de 170 mg (2,1.$10^{-3}$ mole) d'oxyde de zinc (ZnO) dans 4 ml de toluène anhydre et 4 ml d'acétonitrile est maintenue à 50 °C sous agitation, sous atmosphère inerte en présence de tamis moléculaire (1 nm) pendant 48 h. Le milieu réactionnel est alors filtré sur Célite[R] dans l'acétate d'éthyle puis lavé au moyen d'une solution aqueuse d'acide chlorhydrique 1N, d'eau, d'une solution de soude 1N puis d'une solution saturée de NaCl. La solution obtenue est séchée sur sulfate de sodium et le solvant est évaporé sous pression réduite. Le résidu obtenu est purifié par chromatographie sur gel de silice en éluant avec un mélange hexane/acétate d'éthyle, 2/1 (v/v). On obtient 194 mg (rendement : 35 %) du produit attendu.
F = 145-148 °C

**PREPARATION LXXIX**

**Obtention du 2,3,4-tri-O-acétyl-5-thio-α-D-xylopyranose**

A une solution de 15 g (44,8.$10^{-3}$ mole) de 1,2,3,4-tétra-O-acétyl-5-thio-D-xylopyranose dans 450 ml d'éther, on ajoute 22 ml (0,2 mole) de benzylamine sous atmosphère inerte. Après 7 heures à température ambiante (15-25 °C), le mélange réactionnel est concentré sous pression réduite, le résidu est dissous dans du dichlorométhane, lavé au moyen d'une solution d'acide chlorhydrique 1N, d'une solution saturée de chlorure d'ammonium puis à l'eau. La solution obtenue est séchée sur sulfate de sodium et le solvant est évaporé sous pression réduite. Après purification par chromatographie sur gel de silice en éluant au moyen d'un mélange hexane/acétate d'éthyle, 3/2 (v/v) et cristallisation dans un mélange acétate d'éthyle/hexane, on obtient 6,2 g (rendement : 47 %) du produit attendu dont les caractéristiques physiques sont les suivantes :
F = 115 °C
$[\alpha]_D^{21 °C}$ = + 131 ° (c = 0,34 ; $CHCl_3$)

**PREPARATION LXXX**

**Obtention du trichloracétimidate de 2,3,4-tri-O-acétyl-5-thio-α-D-xylopyranosyle**

A une solution de 1 g (3,42.$10^{-3}$ mole) de 2,3,4-tri-O-acétyl-5-thio-α-D-xylopyranose dans 10 ml de dichlorométhane, on ajoute 1,5 ml (15.$10^{-3}$ mole) de trichloracétonitrile et 70 mg de NaH (2,3.$10^{-3}$ mole de NaH à 80 % ). Après 4 h à température ambiante, le milieu réactionnel est filtré sur silice dans le dichlorométhane puis purifié par chromatographie sur gel de silice en éluant au moyen d'un mélange hexane/acétate d'éthyle, 3/1 (v/v). Après cristallisation dans un mélange d'acétate d'éthyle/hexane, on obtient 790 mg (rendement : 53 %) du produit attendu.
F = 110 °C
$[\alpha]_D^{21 °C}$ = + 227 ° (c = 0,42 ; $CHCl_3$)

**PREPARATION LXXXI**

**Obtention du 4-cyanophényl 2,3,4-tri-O-acétyl-5-thio-β-D-xylopyranoside (exemple 22a)**

A une suspension de 250 mg (0,57.$10^{-3}$ mole) de trichloracétimidate de 2,3,4-tri-O-acétyl-5-thio-α-D-xylopyranosyle, de 57 mg (0,48.$10^{-3}$ mole) de 4-hydroxybenzonitrile, de tamis moléculaire (1 nm) dans 10 ml de dichlorométhane, on ajoute, sous atmosphère inerte à -15 °C, 1 ml d'une solution d'éthérate de trifluorure de bore 0,1 M dans le dichlorométhane. On laisse revenir doucement à 0 °C et on neutralise, après 3 h de réaction, avec du bicarbonate de sodium. Le mélange réactionnel est ensuite lavé à l'eau, séché sur sulfate de magnésium $MgSO_4$ et le solvant est évaporé sous pression réduite. Après purification par chromatographie sur gel de silice en éluant au moyen d'un mélange hexane/acétate d'éthyle, 2/1 (v/v), on obtient 150 mg (rendement : 80 %) du produit attendu.
F = 145-148 °C

**PREPARATION LXXXII**

**Obtention du 4-cyanophényl 5-thio-β-D-xylopyranoside (exemple 22)**

A une suspension de 10 g (25,4.$10^{-3}$ mole) de 4-cyanophényl 2,3,4-tri-O-acétyl-5-thio-β-D-xylopyranoside dans 200 ml de méthanol, on ajoute sous atmosphère inerte, 1,5 ml d'une solution de méthylate de sodium dans le méthanol (8 % p/v de Na). Le mélange réactionnel est maintenu sous agitation à température ambiante pendant 30 min, neutralisé par addition de résine Amberlite$^R$ IR 120 $H^+$ et filtré. Après évaporation à sec, le résidu est cristallisé dans le méthanol. On obtient 8,8 g (rendement : 73 %) du produit attendu.
F = 179-186 °C
$[\alpha]_D^{21°C}$ = - 108,6 ° (c = 0,48 ; $CH_3OH$)

**PREPARATION LXXXIII**

**Obtention du 4-nitrophényl 2,3,4-tri-O-acétyl-5-thio-β-D-xylopyranoside (exemple 23a)**

Une suspension de 5,6 g (15,8.$10^{-3}$ mole) de bromure de 2,3,4-tri-0-acétyl-5-thio-α-D-xylopyranosyle, de 2 g (14,3.$10^{-3}$ mole) de 4-nitrophénol, de 4 g (29,3.$10^{-3}$ mole) de chlorure de zinc et de 3,8 g (21,7.$10^{-3}$ mole) d'imidazolate d'argent dans 80 ml de dichlorométhane anhydre est maintenue à 50 °C sous agitation, sous atmosphère inerte à l'abri de la lumière, en présence de tamis moléculaire (400 pm). Après 48 h à cette température, le mélange réactionnel est filtré sur Célite$^R$, lavé au moyen d'une solution aqueuse d'acide chlorhydrique 1N, puis d'une solution de soude 1N et enfin à l'eau et séché sur sulfate de magnésium ($MgSO_4$). Après évaporation à sec, le résidu est purifié par chromatographie sur gel de silice en éluant au moyen d'un mélange hexane/acétate d'éthyle, 3/1 (v/v). Par précipitation dans l'éther, on obtient 1,5 g (rendement : 25 %) du produit attendu.
F = 212 °C
$[\alpha]_D^{21°C}$ = - 78 ° (c = 0,5 ; $CHCl_3$)

**PREPARATION LXXXIV**

**Obtention du 4-nitrophényl 5-thio-β-D-xylopyranoside (exemple 23)**

A une suspension de 1,1 g (2,6.$10^{-3}$ mole) de 4-nitrophényl 2,3,4-tri-O-acétyl-5-thio-β-D-xylopyranoside dans 30 ml de méthanol, on ajoute, sous atmosphère inerte, 0,2 ml d'une solution de méthylate de sodium dans le méthanol (8 % p/v de Na). Le mélange réactionnel est neutralisé après complète solubilisation (2 h) par addition de résine Amberlite$^R$ IR 120 $H^+$ puis filtré. Après évaporation à sec et lyophilisation, on obtient 620 mg (rendement : 79 %) du produit attendu.
F = 130-132 °C
$[\alpha]_D^{21°C}$ = - 77,3 ° (c = 0,49 ; $CH_3OH$)

**PREPARATION LXXXV**

**Obtention du 4-acétylphényl 2,3,4-tri-O-acétyl-5-thio-β-D-xylopyranoside (exemple 24a)**

Selon le mode opératoire décrit dans la préparation LXXXIII, au départ de 2 g (14,7.$10^{-3}$ mole) de 1-(4-hydroxyphényl) éthanone, 2,8 g (16.$10^{-3}$ mole) d'imidazolate d'argent, 5,74 g (16,1.$10^{-3}$ mole) de bromure de 2,3,4-tri-O-acétyl-5-thio-α-D-xylopyranosyle et de 4 g (29,3.$10^{-3}$ mole) de chlorure de zinc dans 100 ml de dichlorométhane, on obtient, après purification par chromatgraphie sur gel de silice en éluant au moyen d'un mélange toluène/acétate d'éthyle, 6/1 (v/v), 0,96 g (rendement : 18 %) du produit attendu.
F = 156 °C
$[\alpha]_D^{21°C}$ = - 77 ° (c = 0,5 ; $CHCl_3$)

**PREPARATION LXXXVI**

**Obtention du 4-acétylphényl 2,3,4-tri-O-acétyl-5-thio-β-D-xylopyranoside (exemple 24a)**

Selon le mode opératoire décrit dans la préparation LXXXI, au départ de 380 mg (0,882.$10^{-3}$ mole) de trichloracétimidate de 2,3,4-tri-O-acétyl-5-thio-α-D-xylopyranosyle, de 100 mg (73,5.$10^{-3}$ mole) de 1-(4-hydroxyphényl)-éthanone et de 1,47 ml d'une solution d'éthérate de trifluorure de bore 0,1 M dans le dichlorométhane, on obtient, après cristallisation dans l'éther, 140 mg (rendement : 47 %) du produit attendu.
F = 156 °C

**PREPARATION LXXXVII**

**Obtention du 4-acétylphényl 5-thio-β-D-xylopyranoside (exemple 24)**

Selon le mode opératoire décrit dans la préparation LXXXIV, au départ de 0,9 g (2,2.$10^{-3}$ mole) de 4-acétylphényl 2,3,4-tri-O-acétyl-5-thio-β-D-xylopyranoside et 0,8 ml d'une solution de méthylate de sodium dans le méthanol (8 % p/v de Na) dans 50 ml de méthanol pendant 1 h, on obtient, après lyophilisation, 0,55 g (rendement : 88 %) du produit attendu.
F = 195-198 °C
$[\alpha]_D^{21°C}$ = - 84,5 ° (c = 0,49 ; $CH_3OH$)

**PREPARATION LXXXVIII**

**Obtention du 3-acétylphényl 2,3,4-tri-O-acétyl-5-thio-β-D-xylopyranoside (exemple 25a)**

Selon le mode opératoire décrit dans la préparation LXXXIII, au départ de 3,45 g (25,3.$10^{-3}$ mole) de 1-(3-hydroxyphényl)-éthanone, 6 g (16,9.$10^{-3}$ mole) de bromure de 2,3,4-tri-O-acétyl-5-thio-D-xylopyranosyle, 3 g (17.$10^{-3}$ mole) d'imidazolate d'argent et 4,6 g (33,7.$10^{-3}$ mole) de chlorure de zinc dans 90 ml de dichlorométhane et 30 ml d'acétonitrile, on obtient, après purification par chromatographie sur gel de silice en éluant au moyen d'un mélange toluène/acétate d'éthyle, 6/1 (v/v) et cristallisation dans l'éther, 0,96 g (rendement : 14 %) du produit attendu.
F = 150-153 °C
$[\alpha]_D^{21°C}$ = - 81,5 ° (c = 0,5 ; $CHCl_3$)

**PREPARATION LXXXIX**

**Obtention du 3-acétylphényl 5-thio-β-D-xylopyranoside (exemple 25)**

Selon le mode opératoire décrit dans la préparation LXXXIV, au départ de 1,36 g (3,3.$10^{-3}$ mole) de 3-acétylphényl 2,3,4-tri-O-acétyl-5-thio-β-D-xylopyranoside et 0,2 ml d'une solution de méthylate de sodium dans le méthanol (8 % p/v de Na) dans 50 ml de méthanol pendant 30 min, on obtient, après cristallisation dans un mélange éthanol/éther, 0,8 g (rendement : 85 %) du produit attendu.
F = 166-174 °C
$[\alpha]_D^{21°C}$ = - 109 ° (c = 0,42 ; $CH_3OH$)

**PREPARATION XC**

**Obtention du 2-cyanophényl 2,3,4-tri-O-acétyl-5-thio-β-D-xylopyranoside (exemple 26a)**

Selon le mode opératoire décrit dans la préparation LXXXIII, au départ de 2 g (16,8.$10^{-3}$ mole) de 2-hydroxybenzonitrile, 4,4 g (25,1.$10^{-3}$ mole) d'imidazolate d'argent, 6,5 g (18,3.$10^{-3}$ mole) de bromure de 2,3,4-tri-O-acétyl-5-thio-α-D-xylopyranosyle et 4,6 g (33,6.$10^{-3}$ mole) de chlorure de zinc dans 80 ml de dichlorométhane, on obtient, après purification par chromatographie sur gel de silice en éluant au moyen d'un mélange toluène/acétate d'éthyle, 6/1 (v/v) et précipitation dans l'éther, 1,32 g (rendement : 20 %) du produit attendu.
F = 176 °C
$[\alpha]_D^{21°C}$ = - 160 ° (c = 0,45 ; $CHCl_3$)

**PREPARATION XCI**

**Obtention du 2-cyanophényl 5-thio-$\beta$-D-xylopyranoside (exemple 26)**

Selon le mode opératoire décrit dans la préparation LXXXIV, au départ de 1,26 g ($3,2.10^{-3}$ mole) de 2-cyanophényl 2,3,4-tri-O-acétyl-5-thio-$\beta$-D-xylopyranoside et 0,2 ml d'une solution de méthylate de sodium dans le méthanol (8 % p/v de Na) dans 70 ml de méthanol pendant 30 min, on obtient, après précipitation dans l'éther et lyophilisation, 0,75 g (rendement : 88 %) du produit attendu.

F = 130-132 °C

$[\alpha]_D^{21°C}$ = - 68,8° (c = 0,485 ; $CH_3OH$)

**PREPARATION XCII**

**Obtention du 3-cyanophényl 2,3,4-tri-O-acétyl-5-thio-$\beta$-D-xylopyranoside (exemple 27a)**

Selon le mode opératoire décrit dans la préparation LXXXIII, au départ de 2 g ($16,8.10^{-3}$ mole) de 3-hydroxybenzonitrile, 2,9 mg ($16,5.10^{-3}$ mole) d'imidazolate d'argent, 6,5 g ($18,3.10^{-3}$ mole) de bromure de 2,3,4-tri-O-acétyl-5-thio-$\alpha$-D-xylopyranosyle et 4,6 g ($33,6.10^{-3}$ mole) de chlorure de zinc dans 80 ml de dichlorométhane, on obtient, après purification par chromatographie sur gel de silice en éluant au moyen d'un mélange hexane/acétate d'éthyle, 3/1 (v/v) et précipitation dans l'éther, 2,2 g (rendement : 33 %) du produit attendu.

F = 148-151 °C

$[\alpha]_D^{21°C}$ = - 82° (c = 0,31 ; $CHCl_3$)

**PREPARATION XCIII**

**Obtention du 3-cyanophényl 2,3,4-tri-O-acétyl-5-thio-$\beta$-D-xylopyranoside (exemple 27a)**

Selon le mode opératoire décrit dans la préparation LXXVIII, au départ de 0,5 g ($1,4.10^{-3}$ mole) de bromure de 2,3,4-tri-O-acétyl-5-thio-D-xylopyranosyle, de 0,25 g ($2,1.10^{-3}$ mole) de 3-hydroxybenzonitrile, de 170 mg ($2,1.10^{-3}$ mole) d'oxyde de zinc (ZnO) dans 4 ml de toluène anhydre et 4 ml d'acétonitrile, on obtient, après purification par chromatographie en éluant sur gel de silice au moyen d'un mélange hexane/acétate d'éthyle, 2/1 (v/v) et précipitation dans l'éther, 138 mg (rendement : 25 %) du produit attendu.

F = 148-151 °C

**PREPARATION XCIV**

**Obtention du 3-cyanophényl 5-thio-$\beta$-D-xylopyranoside (exemple 27)**

Selon le mode opératoire décrit dans la préparation LXXXIV, au départ de 2,12 g ($5,4.10^{-3}$ mole) de 3-cyanophényl 2,3,4-tri-O-acétyl-5-thio-$\beta$-D-xylopyranoside et 0,2 ml d'une solution de méthylate de sodium dans le méthanol (8 % p/v de Na) dans 60 ml de méthanol pendant 30 min, on obtient, après précipitation dans l'éther et lyophilisation, 1,22 g (rendement : 85 %) du produit attendu.

F = 130-135 °C

$[\alpha]_D^{21°C}$ = - 107,4° (c = 0,47 ; $CH_3OH$)

**PREPARATION XCV**

**Obtention du 2-nitrophényl 2,3,4-tri-O-acétyl-5-thio-$\beta$-D-xylopyranoside (exemple 28a)**

Selon le mode opératoire décrit dans la préparation LXXXIII, au départ de 2 g ($14,4.10^{-3}$ mole) de 2-nitrophénol, 2,5 g ($14,2.10^{-3}$ mole) d'imidazolate d'argent, 6 g ($15,7.10^{-3}$ mole) de bromure de 2,3,4-tri-O-acétyl-5-thio-$\alpha$-D-xylopyranosyle et 4 g ($29,3.10^{-3}$ mole) de chlorure de zinc dans 80 ml de dichlorométhane, on obtient, après purification par chromatographie sur gel de silice en éluant au moyen d'un mélange hexane/acétate d'éthyle, 2/1 (v/v) et précipitation dans l'éther, 0,91 g (rendement : 15 %) du produit attendu.

F = 148 °C

$[\alpha]_D^{21°C}$ = - 102,8° (c = 0,40 ; $CHCl_3$)

**PREPARATION XCVI**

**Obtention du 2-nitrophényl 5-thio-β-D-xylopyranoside (exemple 28)**

Selon le mode opératoire décrit dans la préparation LXXXIV, au départ de 0,85 g (2,05.$10^{-3}$ mole) de 2-nitrophényl 2,3,4-tri-O-acétyl-5-thio-β-D-xylopyranoside et 0,1 ml d'une solution de méthylate de sodium dans le méthanol (8 % p/v de Na) dans 50 ml de méthanol pendant 30 min, on obtient, après précipitation dans l'éther et lyophilisation, 0,4 g (rendement : 68 %) du produit attendu.
F = 137-140 °C
$[\alpha]_D^{21°C}$ = - 117 ° (c = 0,35 ; $CH_3OH$)

**PREPARATION XCVII**

**Obtention du 2-acétylphényl 2,3,4-tri-O-acétyl-5-thio-β-D-xylopyranoside (exemple 29a)**

Selon le mode opératoire décrit dans la préparation LXXXIII, au départ de 3,45 g (25,3.$10^{-3}$ mole) de 1-(2-hydroxyphényl) éthanone, 6 g (16,9.$10^{-3}$ mole) de bromure de 2,3,4-tri-O-acétyl-5-thio-D-xylopyranosyle, 3 g (17.$10^{-3}$ mole) d'imidazolate d'argent et 4,6 g (33,7.$10^{-3}$ mole) de chlorure de zinc dans 90 ml de dichlorométhane et 30 ml d'acétonitrile, on obtient, après purification par chromatographie sur gel de silice en éluant au moyen d'un mélange toluène/acétate d'éthyle, 6/1 (v/v) et cristallisation dans l'éther, 0,92 g (rendement : 13,5 %) du produit attendu.
F = 112 °C
$[\alpha]_D^{21°C}$ = - 100 ° (c = 0,42 ; $CHCl_3$)

**PREPARATION XCVIII**

**Obtention du 2-acétylphényl 5-thio-β-D-xylopyranoside (exemple 29)**

Selon le mode opératoire décrit dans la préparation LXXXIV, au départ de 0,88 g (2,1.$10^{-3}$ mole) de 2-acétylphényl 2,3,4-tri-O-acétyl-5-thio-β-D-xylopyranoside et 0,1 ml d'une solution de méthylate de sodium dans le méthanol (8 % p/v de Na) dans 50 ml de méthanol pendant 30 min, on obtient, après purification par chromatographie sur gel de silice en éluant au moyen d'un mélange chloroforme/méthanol, 12/1 (v/v) et lyophilisation, 0,51 g (rendement : 84 %) du produit attendu.
F = 102-105 °C
$[\alpha]_D^{21°C}$ = - 90 ° (c = 0,44 ; $CH_3OH$)

**PREPARATION IC**

**Obtention du 2-(6-cyanonaphtalényl) 2,3,4-tri-O-acétyl-5-thio-β-D-xylopyranoside (exemple 30a)**

Selon le mode opératoire décrit dans la préparation LXXXIII, au départ de 1,69 g (10.$10^{-3}$ mole) de 6-hydroxy-2-naphtalènecarbonitrile, 1,75 g (10.$10^{-3}$ mole) d'imidazolate d'argent, 3,87 g (11,1.$10^{-3}$ mole) de bromure de 2,3,4-tri-O-acétyl-5-thio-α-D-xylopyranosyle et 2,7 g (19,7.$10^{-3}$ mole) de chlorure de zinc dans 80 ml de dichlorométhane, on obtient, après purification par chromatgraphie sur gel de silice en éluant au moyen d'un mélange hexane/acétate d'éthyle, 3/1 (v/v) et précipitation dans l'éther, 0,72 g (rendement : 16 %) du produit attendu.
F = 194 °C
$[\alpha]_D^{21°C}$ = - 57,4 ° (c = 0,5 ; $CHCl_3$)

**PREPARATION C**

**Obtention du 2-(6-cyanonaphtalényl) 5-thio-β-D-xylopyranoside (exemple 30)**

Selon le mode opératoire décrit dans la préparation LXXXIV, au départ de 0,92 g (2,1.$10^{-3}$ mole) de 2-(6-cyanonaphtalényl) 2,3,4-tri-O-acétyl-5-thio-β-D-xylopyranoside et 0,2 ml d'une solution de méthylate de sodium dans le méthanol (8 % p/v de Na) dans 40 ml de méthanol et un ajout de tétrahydrofuranne jusqu'à solubilité, on obtient, après précipitation dans l'éther et cristallisation dans le méthanol, 0,59 g (rendement : 90 %) du produit attendu.

F = 209-214 °C
$[\alpha]_D^{21°C}$ = - 83 ° (c = 0,2 ; $CH_3OH$)

**PREPARATION CI**

**Obtention du 2-(1-cyanonaphtalényl) 2,3,4-tri-O-acétyl-5-thio-β-D-xylopyranoside (exemple 31a)**

Selon le mode opératoire décrit dans la préparation LXXXIII, au départ de 5 g ($29,5.10^{-3}$ mole) de 2-hydroxy-1-naphtalènecarbonitrile, 4,8 g ($32,7.10^{-3}$ mole) d'imidazolate d'argent, 10,5 g ($29,5.10^{-3}$ mole) de bromure de 2,3,4-tri-O-acétyl-5-thio-α-D-xylopyranosyle et 12 g ($88.10^{-3}$ mole) de chlorure de zinc dans 200 ml de dichlorométhane, on obtient, après purification par chromatographie sur colonne de silice en éluant au moyen d'un mélange hexane/acétate d'éthyle, 7/3 (v/v) puis lavage à l'éther, 3,85 g (rendement : 29,4 %) du produit attendu.
F = 192 °C (décomposition)
$[\alpha]_D^{24°C}$ = - 141,6 ° (c = 0,3 ; $CHCl_3$)

**PREPARATION CII**

**Obtention du 2-(1-cyanonaphtalényl 5-thio-β-D-xylopyranoside (exemple 31)**

Selon le mode opératoire décrit dans la préparation LXXXIV, au départ de 3 g ($6,76.10^{-3}$ mole) de 2-(1-cyanonaphtalényl) 2,3,4-tri-O-acétyl-5-thio-β-D-xylopyranoside et 1,2 ml d'une solution de méthylate de sodium dans le méthanol (8 % p/v de Na) dans 70 ml de méthanol pendant 3,5 h, on obtient, après recristallisation dans un mélange méthanol/eau, 5/1 (v/v), 1,30 g (rendement : 59 %) du produit attendu.
F = 163-164 °C
$[\alpha]_D^{24°C}$ = + 13 ° (c = 0,29 ; $CH_3OH$)

**PREPARATION CIII**

**Obtention du 4-acétamidophényl 2,3,4-tri-O-acétyl-5-thio-β-D-xylopyranoside (exemple 32a)**

A une solution de 150 mg ($0,36.10^{-3}$ mole) de 4-nitrophényl 2,3,4-tri-O-acétyl-5-thio-β-D-xylopyranoside dans l'anhydride acétique sous atmosphère d'azote, 30 mg de charbon palladié à 10 % sont ajoutés puis le mélange réactionnel obtenu est maintenu sous pression d'hydrogène ($3,5.10^5$ Pa) à 50 °C pendant 15 heures. Après filtration, le mélange est évaporé à sec sous pression réduite et le résidu obtenu est purifié par chromatographie en éluant au moyen d'un mélange chlorure de méthylène/méthanol, 98/2 (v/v). On obtient 80 mg (rendement : 51 %) du produit attendu.
F = 166 °C
$[\alpha]_D^{25°C}$ = - 34 ° (c = 0,25 ; $CHCl_3$)

**PREPARATION CIV**

**Obtention du 4-acétamidophényl 5-thio-β-D-xylopyranoside (exemple 32)**

Selon le mode opératoire décrit dans la préparation LXXXIV, au départ de 1,2 g ($2,8.10^{-3}$ mole) de 4-acétamidophényl 2,3,4-tri-O-acétyl-5-thio-β-D-xylopyranoside et 70 µl d'une solution de méthylate de sodium dans le méthanol (8 % p/v de Na) dans 100 $cm^3$ de méthanol, on obtient, après purification par "flash chromatography" en éluant au moyen d'un mélange chlorure de méthylène/méthanol, 93/7 (v/v), 0,7 g (rendement : 83 %) du produit attendu.
F = 238 °C
$[\alpha]_D^{25°C}$ = - 48,3 ° (c = 0,145 ; DMSO)
De façon non limitative, on a consigné les composés suivant l'invention dans les tableaux I et I bis donnés ci-après, le tableau I concernant les composés de formule I de structure phénylthioxyloside et le tableau I bis concernant les composés de formule I de structure naphtalénylthioxyloside.

L'activité anti-thrombotique des produits selon l'invention a été mise en évidence selon le protocole opératoire de thrombose veineuse suivant :

On réalise une stase veineuse sous hypercoagulation selon la technique décrite par WESSLER et al. (J. Applied Physiol. 1959, p. 943-946). L'agent hypercoagulant utilisé est, comme dans la technique décrite par

J. HAUPMAN et al. (Thrombosis and Haemostasis 43 (2) 1980, p. 118), une solution de facteur X activé (Xa) fourni par la société dite Flow Laboratories (71 Knat pour 12,5 ml de sérum physiologique).

L'étude est réalisée sur des rats mâles Wistar, non à jeûn, de 25O à 28O g répartis en lots de 10 animaux chacun. Les produits à tester sont administrés per os, en suspension dans le PEG 400. Une thrombose est induite 4 heures après ce traitement et le thrombus formé est prélevé et pesé.

Les résultats obtenus aux doses de 3 mg/kg ou 12,5 mg/kg p.o. ont été consignés dans le tableau II. On a également consigné dans ce tableau les résultats obtenus avec les produits connus de l'art antérieur précité.

Les produits selon l'invention présentent une activité antithrombotique veineuse nettement supérieure à l'activité des produits connus de l'art antérieur.

TABLEAU I

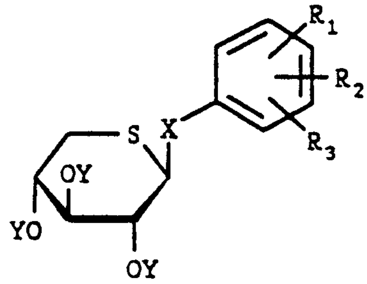

(I)

| Ex | X | Y | R1 | R2 | R3 |
|---|---|---|---|---|---|
| 1a | S | $-COCH_3$ | 4-CN | -H | -H |
| 1 | S | -H | 4-CN | -H | -H |
| 2a | S | $-COCH_3$ | $4-NO_2$ | -H | -H |
| 2 | S | -H | $4-NO_2$ | -H | -H |
| 4a | S | $-COCH_3$ | $4-CF_3$ | -H | -H |
| 4 | S | -H | $4-CF_3$ | -H | -H |
| 5a | S | $-COCH_3$ | 3-CN | -H | -H |
| 5 | S | $-COCH_3$ | 3-CN | -H | -H |
| 6a | S | $-COCH_3$ | 2-CN | -H | -H |
| 6 | S | -H | 2-CN | -H | -H |
| 7a | S | $-COCH_3$ | $2-NO_2$ | -H | -H |
| 7 | S | -H | $2-NO_2$ | -H | -H |
| 9a | S | $-COCH_3$ | -H | -H | -H |
| 9 | S | -H | -H | -H | -H |
| 10a | S | $-COCH_3$ | $3-OCH_3$ | $4-OCH_3$ | $5-OCH_3$ |
| 10 | S | -H | $3-OCH_3$ | $4-OCH_3$ | $5-OCH_3$ |

## TABLEAU I (suite)

| Ex | X | Y | R1 | R2 | R3 |
|---|---|---|---|---|---|
| 11a | S | $-COCH_3$ | $4-COCH_3$ | -H | -H |
| 11 | S | -H | $4-COCH_3$ | -H | -H |
| 12a | S | $-COCH_3$ | $3-NO_2$ | -H | -H |
| 12 | S | -H | $3-NO_2$ | -H | -H |
| 13a | S | $-COCH_3$ | $2-CF_3$ | -H | -H |
| 13 | S | -H | $2-CF_3$ | -H | -H |
| 14a | S | $-COCH_3$ | 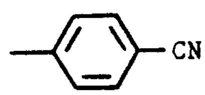 | -H | -H |
| 14 | S | -H | 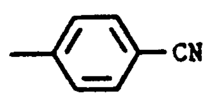 | -H | -H |
| 15a | S | $-COCH_3$ | $3-CF_3$ | $5-CF_3$ | -H |
| 15 | S | -H | $3-CF_3$ | $5-CF_3$ | -H |
| 16a | S | $-COCH_3$ | 2-CN | 4-CN | -H |
| 16 | S | -H | 2-CN | 4-CN | -H |
| 17a | S | $-COCH_3$ | 2-CN | 4-CN | 6-CN |
| 17 | S | -H | 2-CN | 4-CN | 6-CN |
| 18 | S | -H | $4-NH_2$ | -H | -H |
| 19a | S | $-COCH_3$ | $4-NHCOCH_3$ | -H | -H |
| 19 | S | -H | $4-NHCOCH_3$ | -H | -H |
| 20a | S | $-COCH_3$ | $4-COCF_3$ | -H | -H |
| 20 | S | -H | $4-COCF_3$ | -H | -H |

TABLEAU I (fin)

| Ex | X | Y | R1 | R2 | R3 |
|---|---|---|---|---|---|
| 21 | S | -H | 3-$NH_2$ | -H | -H |
| 22a | O | -$COCH_3$ | 4-CN | -H | -H |
| 22 | O | -H | 4-CN | -H | -H |
| 23a | O | -$COCH_3$ | 4-$NO_2$ | -H | -H |
| 23 | O | -H | 4-$NO_2$ | -H | -H |
| 24a | O | -$COCH_3$ | 4-$COCH_3$ | -H | -H |
| 24 | O | -H | 4-$COCH_3$ | -H | -H |
| 25a | O | -$COCH_3$ | 3-$COCH_3$ | -H | -H |
| 25 | O | -H | 3-$COCH_3$ | -H | -H |
| 26a | O | -$COCH_3$ | 2-CN | -H | -H |
| 26 | O | -H | 2-CN | -H | -H |
| 27a | O | -$COCH_3$ | 3-CN | -H | -H |
| 27 | O | -H | 3-CN | -H | -H |
| 28a | O | -$COCH_3$ | 2-$NO_2$ | -H | -H |
| 28 | O | -H | 2-$NO_2$ | -H | -H |
| 29a | O | -$COCH_3$ | 2-$COCH_3$ | -H | -H |
| 29 | O | -H | 2-$COCH_3$ | -H | -H |
| 32a | O | -$COCH_3$ | 4-$NHCOCH_3$ | -H | -H |
| 32 | O | -H | 4-$NHCOCH_3$ | -H | -H |

TABLEAU I bis

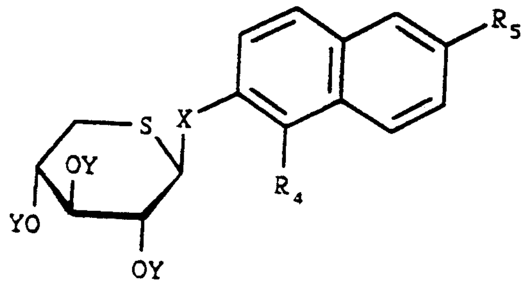

| EX | X | Y | $R_4$ | $R_5$ |
|---|---|---|---|---|
| 8 | S | -H | -H | -CN |
| 8a | S | $-COCH_3$ | -H | -CN |
| 30 | O | -H | -H | -CN |
| 30a | O | $-COCH_3$ | -H | -CN |
| 31 | O | -H | -CN | -H |
| 31a | O | $-COCH_3$ | -CN | -H |

**TABLEAU II**

| Exemple | % d'inhibition à 12,5 mg/kg | % d'inhibition à 3 mg/kg |
|---|---|---|
| 1a | 90 | 30 |
| 1 | 90 | 53 |
| 2 | 78 | 35 |
| 3 | 38 | - |
| 4 | 66 | - |
| 5 | 79,5 | 28 |
| 6 | 88,6 | 35 |
| 7 | 96,7 | 33 |
| 8 | 94,7 | 31 |
| 9 | 11 (1) | - |
| 10a | 62 | - |
| 10 | 36,5 | - |
| 11 | 98,3 | 47 |
| 12 | 69 | - |
| 13 | 23 | - |
| 14 | 86 | 27 |
| 16a | 72,8 | - |
| 16 | 99,5 | 42,5 |
| 17 | 40 | 8 |
| 18 | 93 | 30 |
| 19a | - | 66 |
| 19 | 93 | 61 |
| 20 | - | 49 |

TABLEAU II (fin)

| Exemple | % d'inhibition à 12,5 mg/kg | % d'inhibition à 3 mg/kg |
|---|---|---|
| 21 | - | 31 |
| 22a | - | 66 |
| 22 | - | 57 |
| 23a | - | 44 |
| 23 | 94 | 37,5 |
| 24 | 100 | 55 |
| 25a | - | 52 |
| 25 | - | 44,5 |
| 26 | 96 | 63,5 |
| 27a | - | 57 |
| 27 | 98 | 66 |
| 28 | 94 | 51 |
| 29 | - | 26 |
| 30 | - | 51 |
| 31a | - | 44,5 |
| 31 | - | 28 |
| A | 14 (2) | |
| B | 5,5 | |

A : produit de comparaison décrit à l'exemple 1 de EP-A-0133103

B : produit de comparaison décrit à l'exemple 97 de EP-B-0051023

(1) 82 à 50 mg/kg p.o.

(2) 77 à 50 mg/kg p.o.

**Revendications**

**Revendications pour les Etats contractants suivants : AT, BE, CH, DE, FR, GB, IT, LI, LU, NL, SE**

**1.** Composé oside caractérisé en ce qu'il est choisi parmi l'ensemble comprenant les $\beta$-D-phénylthioxylosides de formule :

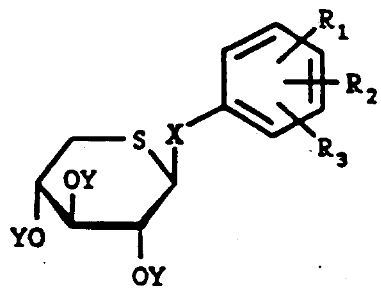

(I)

dans laquelle :
- X représente un atome de soufre ou un atome d'oxygène,
- $R_1$, $R_2$ et $R_3$, identiques ou différents, représentent chacun un atome d'hydrogène, un groupe nitro, un groupe cyano, un groupe -CO-R (où R représente un groupe alkyle en $C_1$-$C_4$ ou un groupe trifluorométhyle), un groupe amino, un groupe acétamido ($NHCOCH_3$), un groupe alkoxy en $C_1$-$C_4$, un groupe trifluorométhyle, un groupe phényle substitué par un ou plusieurs groupes cyano, nitro ou trifluorométhyle, $R_1$ et $R_2$, considérés ensemble, pouvant former avec le groupe phényle auquel ils sont liés un groupe $\beta$-naphtalényle éventuellement substitué par un ou plusieurs groupes cyano, nitro ou trifluorométhyle ; et,
- Y représente l'atome d'hydrogène ou un groupe acyle aliphatique.

**2.** Composé oside selon la revendication 1, caractérisé en ce que :
- X représente un atome de soufre ou un atome d'oxygène,
- $R_1$ représente l'atome d'hydrogène,
- l'un au moins des $R_2$ et $R_3$ représente un groupe cyano ; et,
- Y représente l'atome d'hydrogène ou un groupe acyle aliphatique en $C_2$-$C_5$.

**3.** Composé oside selon l'une quelconque des revendications 1 et 2, caractérisé en ce que le groupe acyle aliphatique Y représente le groupe $CH_3CO$.

**4.** 4-cyanophényl 5-thio-$\beta$-D-xylopyranoside.

**5.** 4-acétylphényl 5-thio-$\beta$-D-xylopyranoside.

**6.** 3-cyanophényl 5-thio-$\beta$-D-xylopyranoside.

**7.** 2-nitrophényl 5-thio-$\beta$-D-xylopyranoside.

**8.** 4-acétylphényl 2,3,4-tri-O-acétyl-5-thio-$\beta$-D-xylopyranoside.

**9.** 2-cyanophényl 5-thio-$\beta$-D-xylopyranoside.

**10.** 4-cyanophényl 1,5-dithio-$\beta$-D-xylopyranoside.

**11.** 2,4-dicyanophényl 1,5-dithio-$\beta$-D-xylopyranoside.

**12.** 4-cyanophényl 2,3,4-tri-O-acétyl-1,5-dithio-$\beta$-D-xylopyranoside.

**13.** 4-acétamidophényl 1,5-dithio-$\beta$-D-xylopyranoside.

**14.** Composition thérapeutique caractérisée en ce qu'elle renferme, en association avec un excipient physiologiquement acceptable, au moins un composé oside selon l'une quelconque des revendications 1 à 13.

**15.** Utilisation d'une substance selon l'une quelconque des revendications 1 à 13, pour l'obtention d'un médicament antithrombotique destiné à une utilisation thérapeutique vis-à-vis des troubles de la circulation veineuse.

**16.** Procédé de préparation d'un β-D-phényl-thioxyloside de formule I selon la revendication 1 caractérisé en ce que :

1°) on fait réagir un composé de formule :

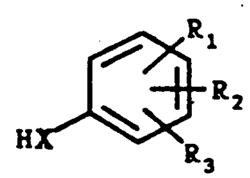

(II)

où X, $R_1$, $R_2$ et $R_3$ sont définis comme indiqué dans la revendication 1,
avec un dérivé du thioxylose choisi parmi l'ensemble comprenant :

(i) les halogénures d'acylthioxylosyle de formule :

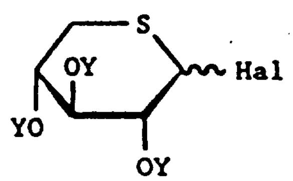

(III)

(ii) les thioxyloses peracylés de formule :

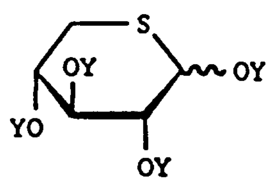

(IV)

(iii) les trichloracétimidates d'acylthioxylosyle de formule :

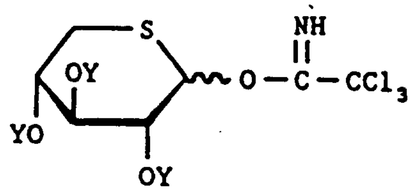

(V)

dans lesquelles Hal représente Cl ou Br et Y représente un groupe acyle aliphatique,
dans un solvant inerte, à raison de 1 mole de II pour environ 0,6 à 1,2 moles de composé III, IV ou V, en présence d'un accepteur d'acide et/ou d'un acide de Lewis et,
2°) si nécessaire, on procède à une réaction de désacylation à une température comprise entre 0°C et la température de reflux du milieu réactionnel, dans un alcool inférieur en $C_1$-$C_4$ en présence

d'un alcoolate métallique, pour obtenir un composé de formule I où Y est H.

**17.** Halogénures d'acyl-β-D-thioxylosyle de formule :

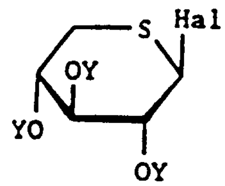

(III')

où Hal représente un atome de brome et Y représente un groupe acyle aliphatique comprenant 2 à 5 atomes de carbone.

**18.** Trichloracétimidates d'acylthioxylosyle de formule :

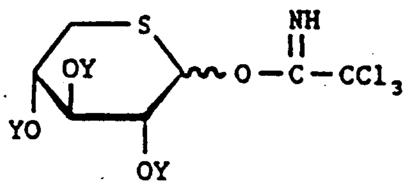

(V)

où Y représente un groupe acyle aliphatique comprenant 2 à 5 atomes de carbone.

**19.** Utilisation d'halogénures d'acylthioxylosyle de formule III'selon la revendication 17 en tant qu'intermédiaires de synthèse dans la préparation des composés de formule I.

**20.** Utilisation de tricloracétimidates d'acylthioxylosyle de formule V selon la revendication 18 en tant qu'intermédiaires de synthèse dans la préparation des composés de formule I.

**21.** Produit intermédiaire nouveau intervenant dans la synthèse des β-D-phényl-thioxylosides de formule I où X représente l'atome de soufre selon la revendication 1, caractérisé en ce qu'il est choisi parmi l'ensemble constitué par les
6-mercapto-2-napthalènecarbonitrile et
3,5-dicyano-2-mercapto-benzonitrile.

**22.** Produit intermédiaire nouveau intervenant dans la synthèse des β-D-phényl-thioxylosides de formule I où X représente l'atome de soufre selon la revendication 1, caractérisé en ce qu'il est choisi parmi l'ensemble constitué par les
diméthylthiocarbamate de O-2-(6-cyanonaphtalényle),
diméthylthiocarbamate de O-3,5-bis(trifluorométhyl)phényle,
diméthylthiocarbamate de O-2,4,6-tricyanophényle,
diméthylthiocarbamate de S-2-(6-cyanonaphtalényle),
diméthylthiocarbamate de S-3,5-bis(trifluorométhyl)phényle, et
diméthylthiocarbamate de S-2,4,6-tricyanophényle.

**Revendications pour les Etats contractants suivants : ES, GR**

**1.** Procédé pour la préparation d'un composé oside choisi parmi l'ensemble comprenant les β-D-phénylthioxylosides de formule :

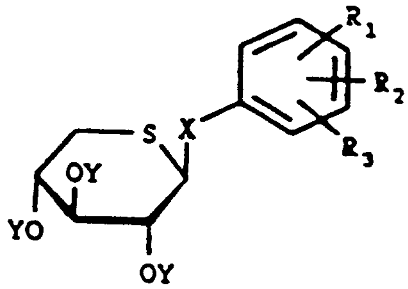

(I)

dans laquelle :

- X représente un atome de soufre ou un atome d'oxygène,
- $R_1$, $R_2$ et $R_3$, identiques ou différents, représentent chacun un atome d'hydrogène, un groupe nitro, un groupe cyano, un groupe -CO-R (où R représente un groupe alkyle en $C_1$-$C_4$ ou un groupe trifluorométhyle), un groupe amino, un groupe acétamido ($NHCOCH_3$), un groupe alkoxy en $C_1$-$C_4$, un groupe trifluorométhyle, un groupe phényle substitué par un ou plusieurs groupes cyano, nitro ou trifluorométhyle, $R_1$ et $R_2$, considérés ensemble, pouvant former avec le groupe phényle auquel ils sont liés un groupe $\beta$-naphtalényle éventuellement substitué par un ou plusieurs groupes cyano, nitro ou trifluorométhyle ; et,
- Y représente l'atome d'hydrogène ou un groupe acyle aliphatique,

ledit procédé étant caractérisé en ce que :

1°) on fait réagir un composé de formule :

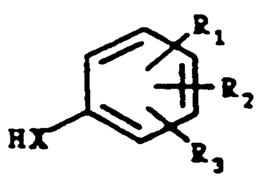

(II)

où X, $R_1$, $R_2$ et $R_3$ sont définis corne ci-dessus.

avec un dérivé du thioxylose choisi parmi l'ensemble comprenant :

(i) les halogénures d'acylthioxylosyle de formule :

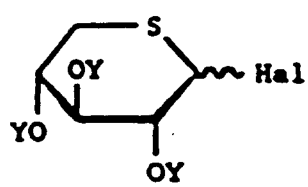

(III)

(ii) les thioxyloses peracylés de formule :

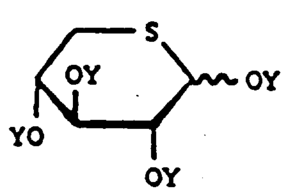

(IV)

(iii) les trichloracétimidates d'acylthioxylosyle de formule :

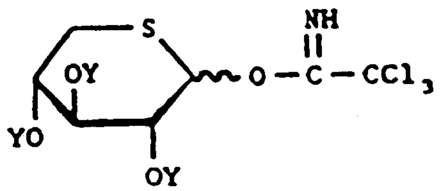

(V)

dans lesquelles Hal représente Cl ou Br et Y représente un groupe acyle aliphatique,
dans un solvant inerte, à raison de 1 mole de II pour environ 0,6 à 1,2 moles de composé III, IV ou V, en présence d'un accepteur d'acide et/ou d'un acide de Lewis et,
2°) si nécessaire, on procède à une réaction de désacylation à une température comprise entre 0°C et la température' de reflux du milieu réactionnel, dans un alcool inférieur en $C_1$-$C_4$ en présence d'un alcoolate métallique, pour obtenir un composé de formule I où Y est H.

**2.** Procédé selon la revendication 1, caractérisé en ce que :
- X représente un atome de soufre ou un atome d'oxygène,
- $R_1$ représente l'atome d'hydrogène,
- l'un au moins des $R_2$ et $R_3$ représente un groupe cyano ; et,
- Y représente l'atome d'hydrogène ou un groupe acyle aliphatique en $C_2$-$C_5$.

**3.** Procédé selon l'une quelconque des revendications 1 et 2, caractérisé en ce que le groupe acyle aliphatique Y représente le groupe $CH_3CO$.

**4.** Procédé selon la revendication 1 pour la préparation d'un composé de formule I où $R_1$ est 4-CN, X est O, et Y = $R_2$ = $R_3$ = H.

**5.** Procédé selon la revendication 1 pour la préparation d'un composé de formule I où $R_1$ est 4-$CH_3CO$, X est O , et Y = $R_2$ = $R_3$ = H.

**6.** Procédé selon la revendication 1 pour la préparation d'un composé de formule I où $R_1$ est 3-CN, X est O, et Y = $R_2$ = $R_3$ = H.

**7.** Procédé selon la revendication 1 pour la préparation d'un composé de formule I où $R_1$ est 2-$NO_2$, X est O , et Y = $R_2$ = $R_3$ = H.

**8.** Procédé selon la revendication 1 pour la préparation d'un composé de formule I où $R_1$ est 4-$COCH_3$, X est O, Y est $CH_3CO$, et $R_2$ = $R_3$ = H.

**9.** Procédé selon la revendication 1 pour la préparation d'un composé de formule I où $R_1$ est 2-CN, X est O , et Y = $R_2$ = $R_3$ = H.

**10.** Procédé selon la revendication 1 pour la préparation d'un composé de formule I où $R_1$ est 4-CN, X est S , et Y = $R_2$ = $R_3$ = H.

**11.** Procédé selon la revendication 1 pour la préparation d'un composé de formule I où $R_1$ est 4-CN, $R_2$ est 2-CN, X est S, et Y = $R_3$ = H.

**12.** Procédé selon la revendication 1 pour la préparation d'un composé de formule I où $R_1$ est 4-CN, X est S, , Y est $CH_3CO$, et $R_2$ = $R_3$ = H.

**13.** Procédé selon la revendication 1 pour la préparation d'un composé de formule I où $R_1$ est 4-$NHCOCH_3$, X est S , et Y = $R_2$ = $R_3$ = H.

## Claims

### Claims for the following Contracting States : AT, BE, CH, DE, FR, GB, IT, LI, LU, NL, SE

**1.** An oside compound selected from the group consisting of the β-D-phenylthioxylosides of the formula

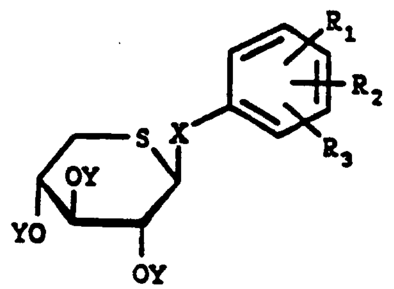

(I)

in which:

- X represents a sulfur atom or an oxygen atom;
- $R_1$, $R_2$ and $R_3$, which are identical or different, each represent a hydrogen atom, a nitro group, a cyano group, a group -CO-R (in which R represents a $C_1$-$C_4$ alkyl group or a trifluoromethyl group), an amino group, an acetamido group ($NHCOCH_3$), a $C_1$-$C_4$ alkoxy group, a trifluoromethyl group or a phenyl group substituted by one or more cyano, nitro or trifluoromethyl groups, it being possible for $R_1$ and $R_2$, taken together, to form, with the phenyl group to which they are bonded, a β-naphthalenyl group which is unsubstituted or substituted by one or more cyano, nitro or trifluoromethyl groups; and
- Y represents the hydrogen atom or an aliphatic acyl group.

**2.** An oside compound according to claim 1 in which:

- X represents a sulfur atom or an oxygen atom;
- $R_1$ represents the hydrogen atom;
- at least one of the radicals $R_2$ and $R_3$ represents a cyano group; and
- Y represents the hydrogen atom or a $C_2$-$C_5$ aliphatic acyl group.

**3.** An oside compound according to claim 1 or claim 2 in which the aliphatic acyl group Y represents the $CH_3CO$ group.

**4.** 4-Cyanophenyl 5-thio-β-D-xylopyranoside.

**5.** 4-Acetylphenyl 5-thio-β-D-xylopyranoside.

**6.** 3-Cyanophenyl 5-thio-β-D-xylopyranoside.

**7.** 2-Nitrophenyl 5-thio-β-D-xylopyranoside.

**8.** 4-Actylphenyl 2,3,4-tri-O-acetyl-5-thio-β-D-xylopyranoside.

**9.** 2-Cyanophenyl 5-thio-β-D-xylopyranoside.

**10.** 4-Cyanophenyl 1,5-dithio-β-D-xylopyranoside.

**11.** 2,4-Dicyanophenyl 1,5-dithio-β-D-xylopyranoside.

**12.** 4-Cyanophenyl 2,3,4-tri-O-acetyl-1,5-dithio-β-D-xylopyranoside.

**13.** 4-Acetamidophenyl 1,5-dithio-β-D-xylopyranoside.

**14.** A therapeutic composition containing, in association with a physiologically acceptable excipient, at least one oside compound according to any one of claims 1 to 13.

**15.** Use of a substance according to any one of claims 1 to 13 in order to obtain an antithrombotic drug for use in therapy vis-a-vis the disorders of the venous circulation.

**16.** A method of preparing a $\beta$-D-phenylthioxyloside of formula I according to claim 1, which comprises:
1°) reacting a compound of the formula

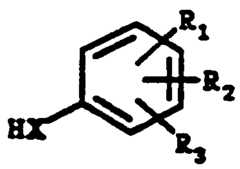

(II)

in which X, $R_1$, $R_2$ and $R_3$ are as indicated in claim 1,
with a thioxylose derivative selected from the group consisting of:
(i) the acylthioxylosyl halides of the formula

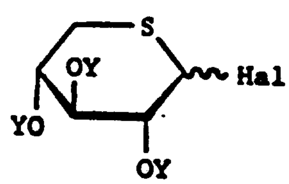

(III)

(ii) the peracylated thioxyloses of the formula

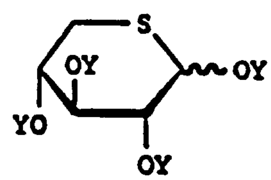

(IV)

and
(iii) the acylthioxylosyl trichloroacetimidates of the formula

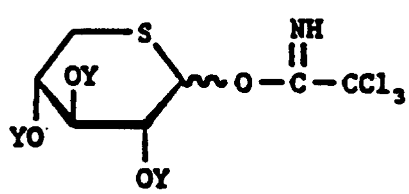

(V)

in which Hal represents Cl or Br and Y represents an acyl group,
in an inert solvent, at a rate of 1 mol of II to about 0.6 to 1.2 mol of compound III, IV or V, in the presence of an acid acceptor and/or a Lewis acid; and
2°) if necessary, carrying out a deacylation reaction at a temperature in the range from 0°C to the reflux temperature of the reaction medium, in a $C_1$-$C_4$ lower alcohol, in the presence of a metal alcoholate, to give a compound of formula I in which Y is H.

**17.** An acyl-β-D-thioxylosyl halide of the formula

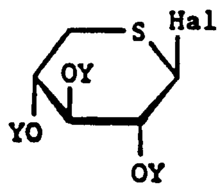

(III')

in which Hal represents a halogen atom selected in particular from the group consisting of the bromine atom and the chlorine atom and Y represents an aliphatic acyl group containing 2 to 5 carbon atoms.

**18.** An acylthioxylosyl trichloroacetimidate of the formula

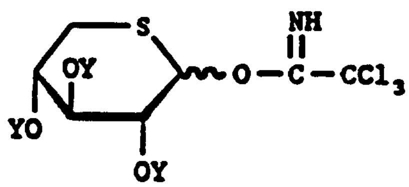

(V)

in which Y represents an aliphatic acyl group containing 2 to 5 carbon atoms.

**19.** The use of an acylthioxylosyl halide of formula III' according to claim 17, as a synthesis intermediate in the preparation of the compounds of formula I.

**20.** The use of an acylthioxylosyl trichloroacetimidate of formula V according to claim 18, as a synthesis intermediate in the preparation of the compounds of formula I.

**21.** A novel intermediate intervening in the synthesis of the β-D-phenylthioxylosides of formula I in which X represents the sulfur atom, according to claim 1, said intermediate being selected from the group consisting of 6-mercaptonaphtalene-2-carbonitrile and 3,5-dicyano-2-mercaptobenzonitrile.

**22.** A novel intermediate intervening in the synthesis of the β-D-phenylthioxylosides of formula I in which X represents the sulfur atom, according to claim 1, said intermediate being selected from the group consisting of O-2-(6-cyanonaphthalenyl) dimethylthiocarbamate, O-3,5-bis-(trifluoromethyl)phenyl dimethylthiocarbamate, O-2,4,6-tricyanophenyl dimethylthiocarbamate, S-2-(6-cyanonaphthalenyl) dimethylthiocarbamate, S-3,5-bis(trifluoromethyl)phenyl dimethylthiocarbamate and S-2,4,6-tricyanophenyl dimethylthiocarbamate.

**Claims for the following Contracting States : ES, GR**

**1.** A method of preparing an oside compound selected from the group consisting of the β-D-phenylthioxylosides of the formula

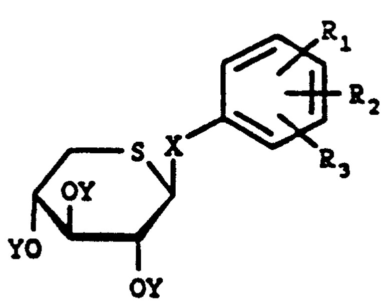

(I)

in which:

- X represents a sulfur atom or an oxygen atom;
- $R_1$, $R_2$ and $R_3$, which are identical or different, each represent a hydrogen atom, a nitro group, a cyano group, a group -CO-R (in which R represents a $C_1$-$C_4$ alkyl group or a trifluoromethyl group), an amino group, an acetamido group ($NHCOCH_3$), a $C_1$-$C_4$ alkoxy group, a trifluoromethyl group or a phenyl group substituted by one or more cyano, nitro or trifluoromethyl groups, it being possible for $R_1$ and $R_2$, taken together, to form, with the phenyl group to which they are bonded, a $\beta$-naphthalenyl group which is unsubstituted or substituted by one or more cyano, nitro or trifluoromethyl groups; and
- Y represents the hydrogen atom or an aliphatic acyl group,

said method comprising:

1°) reacting a compound of the formula

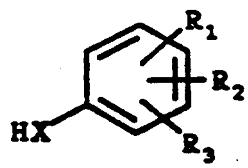

(II)

in which X, $R_1$, $R_2$ and $R_3$ are as defined above,
with a thioxylose derivative selected from the group consisting of:

(i) the acylthioxylosyl halides of the formula

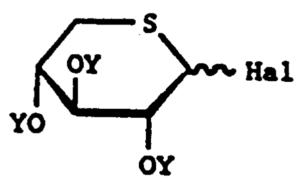

(III)

(ii) the peracylated thioxyloses of the formula

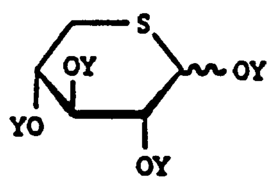

(IV)

and

(iii) the acylthioxylosyl trichloroacetimidates of the formula

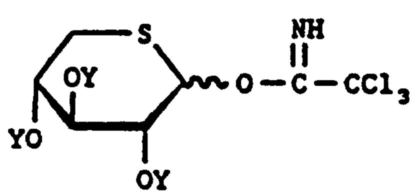

(V)

in which Hal represents Cl or Br and Y represents an aliphatic acyl group,

in an inert solvent, at a rate of 1 mol of II to about 0.6 to 1.2 mol of compound III, IV or V, in the presence of an acid acceptor and/or a Lewis acid; and

2°) if necessary, carrying out a deacylation reaction at a temperature in the range from 0°C to the reflux temperature of the reaction medium, in a $C_1$-$C_4$ lower alcohol, in the presence of a metal

alcoholate, to give a compound of formula I in which Y is H.

2. A method according to claim 1, wherein:
- X represents a sulfur atom or an oxygen atom;
- $R_1$ represents the hydrogen atom;
- at least one of the radicals $R_2$ and $R_3$ represents a cyano group; and
- Y represents the hydrogen atom or a $C_2$-$C_5$ aliphatic acyl group.

3. A method according to claim 1 or claim 2, wherein Y represents the $CH_3CO$ group.

4. A method according to claim 1 for the preparation of a compound of formula I in which $R_1$ is 4-CN, X is O and Y = $R_2$ = $R_3$ = H.

5. A method according to claim 1 for the preparation of a compound of formula I in which $R_1$ is 4-$CH_3CO$, X is O and Y = $R_2$ = $R_3$ = H.

6. A method according to claim 1 for the preparation of a compound of formula I in which $R_1$ is 3-CN, X is O and Y = $R_2$ = $R_3$ = H.

7. A method according to claim 1 for the preparation of a compound of formula I in which $R_1$ is 2-$NO_2$, X is O and Y = $R_2$ = $R_3$ = H.

8. A method according to claim 1 for the preparation of a compound of formula I in which $R_1$ is 4-$COCH_3$, X is O, Y is $CH_3CO$ and $R_2$ = $R_3$ = H.

9. A method according to claim 1 for the preparation of a compound of formula I in which $R_1$ is 2-CN, X is O and Y = $R_2$ = $R_3$ = H.

10. A method according to claim 1 for the preparation of a compound of formula I in which $R_1$ is 4-CN, X is S and Y = $R_2$ = $R_3$ = H.

11. A method according to claim 1 for the preparation of a compound of formula I in which $R_1$ is 4-CN, $R_2$ is 2-CN, X is S and Y = $R_3$ = H.

12. A method according to claim 1 for the preparation of a compound of formula I in which $R_1$ is 4-CN, X is S, Y is $CH_3CO$ and $R_2$ = $R_3$ = H.

13. A method according to claim 1 for the preparation of a compound of formula I in which $R_1$ is 4-$NHCOCH_3$ X is S and Y = $R_2$ = $R_3$ = H.

**Patentansprüche**
**Patentansprüche für folgende Vertragsstaaten : AT, BE, CH, DE, FR, GB, IT, LI, LU, NL, SE**

1. Osid-Verbindung,
**dadurch gekennzeichnet,** daß sie aus der Gruppe ausgewählt ist, die die $\beta$-D-Phenyl-thioxyloside der Formel umfaßt:

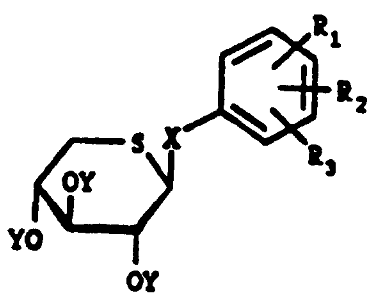

(I)

in der

- X ein Schwefelatom Oder ein Sauerstoffatom darstellt,
- $R_1$, $R_2$ und $R_3$, gleich oder verschieden, jeweils ein Wasserstoffatom, eine Nitrogruppe, eine Cyanogruppe, eine Gruppe -CO-R (worin R eine Alkylgruppe mit 1 bis 4 Kohlenstoffatomen oder eine Trifluormethylgruppe ist), eine Aminogruppe, eine Acetamidogruppe ($NHCOCH_3$), eine Alkoxygruppe mit 1 bis 4 Kohlenstoffatomen, eine Trifluormethylgruppe oder eine Phenylgruppe darstellt, substituiert durch eine oder mehrere Cyanogruppen, Nitrogruppen oder Trifluormethylgruppen, wobei $R_1$ und $R_2$, zusammen betrachtet, mit der Phenylgruppe, an die sie gebunden sind, eine β-Naphtalenyl-Gruppe bilden können, gegebenenfalls substituiert durch eine oder mehrere Cynogruppen, Nitrogruppen oder Trifluormethylgruppen; und
- Y ein Wasserstoffatom oder eine aliphatische Acylgruppe bedeutet.

**2.** Osid-Verbindung nach Anspruch 1,
**dadurch gekennzeichnet,**
daß X ein Schwefelatom oder ein Sauerstoffatom darstellt,

- $R_1$ ein Wasserstoffatom ist,
- mindestens eines von $R_2$ und $R_3$ eine Cyanogruppe bedeutet; und
- Y ein Wasserstoffatom oder eine aliphatische Acylgruppe mit 2 bis 5 Kohlenstoffatomen darstellt.

**3.** Osid-Verbindung nach Anspruch 1 oder 2,
**dadurch gekennzeichnet,**
daß die aliphatische Acylgruppe Y die $CH_3CO$-Gruppe darstellt.

**4.** 4-Cyanophenyl-5-thio-β-D-xylopyranosid

**5.** 4-Acetylphenyl-5-thio-β-D-xylopyanosid

**6.** 3-Cyanophenyl-5-thio-β-D-xylopyranosid

**7.** 2-Nitrophenyl-5-thio-β-D-xylopyranosid

**8.** 4-Acetylphenyl-2, 3, 4-tri-0-acetyl-5-thio-β-D-xylopyranosid

**9.** 2-Cyanophenyl- 5-thio-β-D-xylopyranosid

**10.** 4-Cyanophenyl-1, 5-dithio-β-D-xylopyranosid

**11.** 2, 4-Dicyanophenyl- 1, 5-dithio-β-D-xylopyranosid

**12.** 4-Cyanophenyl-2, 3, 4-tri-0-acetyl-1, 5-dithio-β-D-xylopyranosid

**13.** 4-Acetamidophenyl- 1, 5-dithio-β-D-xylopyranosid.

**14.** Therapeutische Zusammensetzung,
**dadurch gekennzeichnet,**
daß sie in Verbindung mit einem physiologisch verträglichen Bindemittel wenigstens eine Osid-Verbindung gemäß einem der Ansprüche 1 bis 13 aufweist.

**15.** Verwendung einer Substanz gemäß einem der Ansprüche 1 bis 13 zur Herstellung eines antithombotischen Medikaments zur therapeutischen Verwendung für Probleme des venösen Kreislaufs.

**16.** Herstellungsverfahren für ein β-D-Phenyl-Thioxylosid der Formel (I) gemäß Anspruch 1,
**dadurch gekennzeichnet,**
daß:

1) eine Verbindung der Formel:

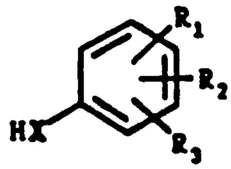

(II)

wobei X, $R_1$, $R_2$ und $R_3$ wie in Anspruch 1 definiert sind, zur Reaktion gebracht wird, mit einem Thioxylose-Derivat ausgewählt aus der Gruppe, die umfaßt:

(i) die Acylthioxylosyl-Halogenide der Formel:

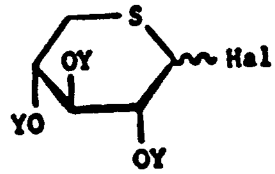

(III)

(ii) die perazylierten Thioxylosen der Formel:

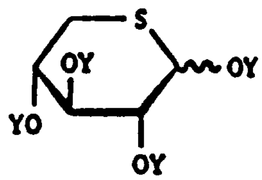

(IV)

(iii) die Acylthioxylosyl-Trichloracetimidate der Formel:

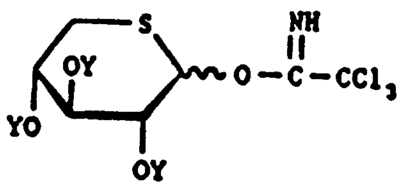

(V)

in denen Hal Chlor oder Brom darstellt und Y eine aliphatische Acylgruppe darstellt,
in einem inerten Lösungsmittel im Verhältnis von einem Mol der Verbindung (II) pro etwa 0,6 bis 1,2 Mol der Verbindung (III), (IV) oder (V), in Anwesenheit eines Säureakzeptors und/oder einer Lewis-Säure, und

2) wenn erforderlich, eine Desacylierungs-Reaktion bei einer Temperatur zwischen 0 °C und der Rückflußtemperatur der Reaktionsmischung in einem niederen Alkohol mit 1 bis 4 Kohlenstoffatomen und in Anwesenheit eines Metall-Alkoholates vornimmt, um eine Verbindung der Formel (I) zu erhalten, in der Y Wasserstoff ist.

**17.** Acyl-β-D-Thiodylosyl-Halogenid der Formel:

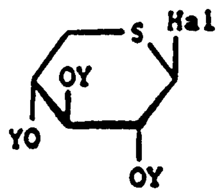

(III')

bei der Hal ein Bromatom darstellt und Y eine aliphatische Acylgruppe darstellt mit 2 bis 5 Kohlenstoffatomen.

**18.** Acylthiodylosyl-Trichloracetimidat der Formel:

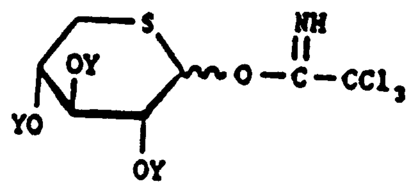

(V)

wobei Y eine aliphatische Acylgruppe darstellt mit 2 bis 5 Kohlenstoffatomen.

**19.** Verwendung eines Acylthioxylosyl-Halogenids der Formel (III') gemäß Anspruch 17 als Zwischenprodukt der Synthese bei der Herstellung der Verbindungen der Formel (I).

**20.** Verwendung von Acylthioxylosyl-Trichloracetimidat der Formel (V) gemäß Anspruch 18 als Zwischenprodukt der Synthese bei der Herstellung der Verbindungen der Formel (I).

**21.** Neues Zwischenprodukt, das bei der Synthese von β-D-Phenyl-Thioxylosiden der Formel (I) auftritt, bei der X ein Schwefelatom gemäß Anspruch 1 darstellt,
**dadurch gekennzeichnet,**
daß es aus der Gruppe ausgewählt ist, die umfaßt:
6-Mercapto-2-Naphtalencarbonitril und
3,5-Dicyano-2-Mercapto-Benzonitril.

**22.** Neues Zwischenprodukt, das bei der Synthese von β-D-Phenyl-Thioxylosiden der Formel (I) auftritt, wobei X ein Schwefelatom gemäß Anspruch 1 darstellt,
**dadurch gekennzeichnet,**
daß es aus der Gruppe ausgewählt ist, die umfaßt:
0-2-(6-Cyanonaphtalenyl)-Dimethylthiocarbamat
0-3,5-bis (Trifluormethyl)-Phenyl-Dimethylthiocarbamat
0-2, 4, 6-Tricyanophenyl-Dimethylthiocarbamat
S-2-(6-Cyanonaphtalenyl)-Dimethylthiocarbamat
S-3, 5-bis(Trifluormethyl)-phenyl-Dimethylthiocarbamat, und
S-2, 4, 6-Tricyanophenyl-Dimethylthiocarbamat.

**Patentansprüche für folgende Vertragsstaaten : ES, GR**

**1.** Verfahren zur Herstellung einer Osid-Verbindung, ausgewählt aus der Gruppe, die die β-D-Phenylthioxyloside der Formel umfaßt:

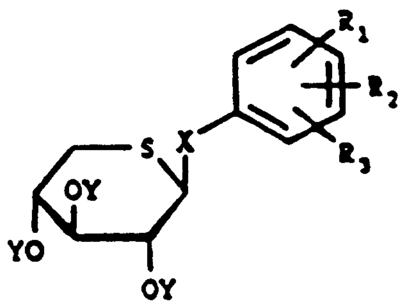

(I)

in der

- X ein Schwefelatom oder ein Sauerstoffatom darstellt,
- $R_1$, $R_2$ und $R_3$, gleich oder verschieden, jeweils ein Wasserstoffatom, eine Nitrogruppe, eine Cyanogruppe, eine Gruppe -CO-R (worin R eine Alkylgruppe mit 1 bis 4 Kohlenstoffatomen oder eine Trifluormethylgruppe ist), eine Aminogruppe, eine Acetamidogruppe ($NHCOCH_3$), eine Alkoxygruppe mit 1 bis 4 Kohlenstoffatomen, eine Trifluormethylgruppe oder eine Phenylgruppe darstellt, substituiert durch eine oder mehrere Cyanogruppen, Nitrogruppen oder Trifluormethylgruppen, wobei $R_1$ und $R_2$, zusammen betrachtet, mit der Phenylgruppe, an die sie gebunden sind, eine $\beta$-Naphthalenyl-Gruppe bilden können, gegebenenfalls substituiert durch eine oder mehrere Cyanogruppen, Nitrogruppen oder Trifluormethylgruppen; und
- Y ein Wasserstoffatom oder eine aliphatische Acylgruppe bedeutet,

**dadurch gekennzeichnet,**

daß man

1) eine Verbindung der Formel

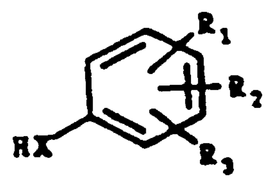

(II)

worin X, $R_1$, $R_2$ und $R_3$ wie oben definiert sind, mit einem Thioxylose-Derivat zur Reaktion bringt, ausgewählt aus der Gruppe, die umfaßt:

(i) die Acylthioxylosyl-Halogenide der Formel

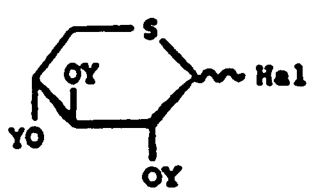

(III)

(ii) die peracylierten Thioxylosen der Formel

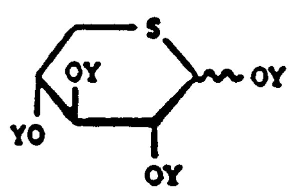

(IV)

(iii) die Acylthioxylosyl-trichloraretimidate der Formel

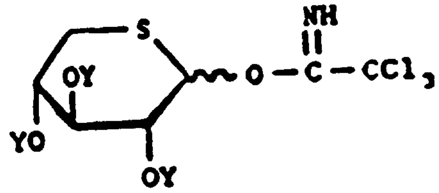

(V)

in denen Hal Cl oder Br darstellt und Y eine aliphatische Acylgruppe ist und zwar in einem inerten Lösungsmittel im Verhältnis von 1 Mol Verbindung (II) pro etwa 0,6 bis 1, 2 Mole der Verbindung (III), (IV) oder (V), in Anwesenheit eines Säureakzeptors und/oder einer Lewis-Säure, und
2) wenn erforderlich, eine Desacylierungs-Reaktion bei einer Temperatur zwischen 0 °C und der Rückflußtemperatur der Reaktionsmischung in einem niederen Alkohol mit 1 bis 4 Kohlenstoffatomen und in Anwesenheit eines Metall-Alkoholates vornimmt, um eine Verbindung der Formel (I) zu erhalten, in der Y Wasserstoff ist.

**2.** Verfahren nach Anspruch 1,
**dadurch gekennzeichnet,**
daß X ein Schwefelatom oder ein Sauerstoffatom darstellt,
- $R_1$ ein Wasserstoffatom ist,
- mindestens eines von $R_2$ und $R_3$ eine Cyanogruppe bedeutet; und
- Y ein Wasserstoffatom oder eine aliphatische Acylgruppe mit 2 bis 5 Kohlenstoffatomen darstellt.

**3.** Verfahren nach irgendeinem der Ansprüche 1 und 2,
**dadurch gekennzeichnet,**
daß die aliphatische Acylgruppe Y die $CH_3CO$-Gruppe darstellt.

**4.** Verfahren nach Anspruch 1 zur Herstellung einer Verbindung der Formel (I) worin $R_1$ 4-CN darstellt, X = O ist und Y = $R_2$ = $R_3$ = Wasserstoff bedeutet.

**5.** Verfahren nach Anspruch 1 zur Herstellung einer Verbindung der Formel (I), worin $R_1$ 4-$CH_3$ CO darstellt, X = O ist und Y = $R_2$ = $R_3$ = Wasserstoff bedeutet.

**6.** Verfahren nach Anspruch 1 zur Herstellung einer Verbindung der Formel (I), worin $R_1$ 3-CN darstellt, X = O ist und Y = $R_2$ = $R_3$ = Wasserstoff bedeutet.

**7.** Verfahren nach Anspruch 1 zur Herstellung einer Verbindung der Formel (I), worin $R_1$ 2-$NO_2$ darstellt, X = O ist und Y = $R_2$ = $R_3$ = Wasserstoff bedeutet.

**8.** Verfahren nach Anspruch 1 zur Herstellung einer Verbindung der Formel (I), worin $R_1$ 4-$COCH_3$ darstellt, X = O ist, Y = $CH_3CO$ ist und $R_2$ = $R_3$ = Wasserstoff bedeutet.

**9.** Verfahren nach Anspruch 1 zur Herstellung einer Verbindung der Formel (I), worin $R_1$ 2-CN darstellt, X = O ist und Y = $R_2$ = $R_3$ = Wasserstoff bedeutet.

**10.** Verfahren nach Anspruch 1 zur Herstellung einer Verbindung der Formel (I), worin $R_1$ 4-CN darstellt, X = S ist und Y = $R_2$ = $R_3$ = Wasserstoff bedeutet,

**11.** Verfahren nach Anspruch 1 zur Herstellung einer Verbindung der Formel (I), worin $R_1$ 4-CN darstellt, $R_2$ 2-CN ist, X = S ist und Y = $R_3$ = Wasserstoff bedeutet.

**12.** Verfahren nach Anspruch 1 zur Herstellung einer Verbindung der Formel (I), worin $R_1$ 4-CN darstellt, X = S ist, Y = $CH_3CO$ ist und $R_2$ = $R_3$ = Wasserstoff bedeutet.

**13.** Verfahren nach Anspruch 1 zur Herstellung einer Verbindung der Formel (I), worin $R_1$ 4-$NHCOCH_3$ darstellt, X = S ist und Y = $R_2$ = $R_3$ = Wasserstoff bedeutet.